(19) Europäisches Patentamt
European Patent Office
Office européen des brevets

(11) Veröffentlichungsnummer: **0 181 837 B1**

# (12) EUROPÄISCHE PATENTSCHRIFT

(45) Veröffentlichungstag der Patentschrift: **02.01.91**

(51) Int. Cl.$^5$: **C 08 G 73/10,** C 07 C 63/33, G 03 F 7/00

(21) Anmeldenummer: **85810527.3**

(22) Anmeldetag: **11.11.85**

## (54) Verfahren zur Herstellung von Filmen und Reliefabbildungen aus Polyimiden.

(30) Priorität: **16.11.84 CH 5486/84**

(43) Veröffentlichungstag der Anmeldung:
**21.05.86 Patentblatt 86/21**

(45) Bekanntmachung des Hinweises auf die Patenterteilung:
**02.01.91 Patentblatt 91/01**

(84) Benannte Vertragsstaaten:
**BE CH DE FR GB IT LI NL SE**

(56) Entgegenhaltungen:
**EP-A-0 047 069**
**EP-A-0 132 221**
**BE-A- 667 307**
**DE-A-1 595 081**
**FR-A-2 053 020**
**US-A-3 702 318**
**US-A-4 131 730**

(73) Patentinhaber: **CIBA-GEIGY AG**
**Klybeckstrasse 141**
**CH-4002 Basel (CH)**

(72) Erfinder: **Pfeifer, Josef, Dr.**
**Brunnmattstrasse 32**
**CH-4106 Therwil (CH)**
Erfinder: **Duthaler, Rudolf, Dr.**
**Streulistrasse 73**
**CH-8032 Zürich (CH)**

**EP 0 181 837 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein Verfahren zur Herstellung von Schutzfilmen oder photographischen Reliefabbildungen unter Verwendung von photovernetzbaren Homo- oder Copolyimiden aus aromatischen, eine Carbonylgruppe enthaltenden Tetracarbonsäuren und Diaminen und gegebenenfalls aromatischen Aminodicarbonsäuren. Die vorliegende Erfindung betrifft ferner neue Tetracarbonsäuren.

Polyimide sind Kunststoff mit wertvollen thermomechanischen Eigenschaften. Auf Grund ihrer hohen Schmelzbereiche können sie jedoch nicht mit den für Thermoplaste üblichen Formgebungsverfahren verarbeitet werden. Es sind daher lösliche Polyimide entwickelt worden, die als Lacke zur Bildung von Ueberzugsschichten mit hoher Wärmebeständigkeit verwendet werden können, vgl. DE—AS—19 62 588 und US—PS—3,787,367. Mit der Entwicklung der Elektronik und Halbeitertechnologie werden an Polyimide z.B. als Isolier- und Schutzfilme hohe Anforderungen an deren Thermostabilität gestellt. Es besteht ferner der Wunsch nach Polyimiden, die photovernetzbar sind und in der Elektronik und Halbeitertechnologie z.B. als Resistmaterial verwendet werden können.

Die im US-Patent 3,702,318 offenbarten Polyimide auf Basis von heterocyclischen Anthrachinonen sind zwar thermisch stabil, aber in organischen Lösungsmitteln unlöslich, da Viskositätsmessungen der betreffenden Polyimide in konzentrierter Schwefelsäure vorgenommen werden.

Desgleichen sind die in der EP—A—0 047 069 beschriebenen Polyimide thermisch stabil. Es wird aber nirgends offenbart, dass Polyimide auf Basis von Ketodinaphthalintetracarbonsäure in organischen Lösungsmitteln löslich und asserdem photovernetzbar sind.

Die im US-Patent 4,131,730 offenbarten Polyimide auf Basis von Aminophthalsäurehydrid sind zwar in organischen Lösungsmitteln im wesentlichen löslich, doch findet sich auch hier nirgends ein Hinweis, dass solche Polyimide photovernetzbar sind, so dass auch hier die Verwendung von Polyimiden als Photoresist dem Fachmann nicht nahegelegt wird.

Es wurde nun gefunden, dass man lösliche Polyimide erhält, wenn man Tetracarbonsäuren verwendet, bei denen aromatische Reste über eine Carbonylgruppe verknüpft sind, und dass man solche Polyimide als Photoresist zur Herstellung von Schutzfilmen oder photographischen Reliefabbildungen verwenden kann.

Gegenstand der Erfindung ist somit ein Verfahren zur Herstellung von Schutzfilm oder photographischen Reliefabbildungen aus Polyimiden, dadurch gekennzeichnet, dass man ein beschichtetes Material aus einem Trägermaterial, auf dem auf mindestens einer Oberfläche eine Schicht eines photovernetzbaren Homo- oder Copolyimids aus mindestens einer aromatischen Tetracarbonsäure und mindestens einem Diamin aufgebracht ist, wobei die Polyimide 0,1 bis 100 Mo-% mindestens eines Strukturelementes der Formel I

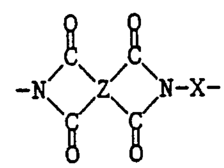 (I)

und 99,9 bis 0 Mol-% mindestens eines Struckturelementes der Formeln II und/oder III

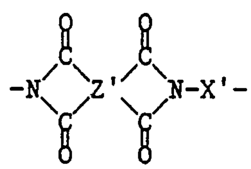 (II),

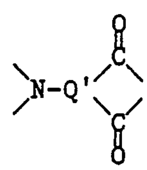 (III)

enthalten, worin

Z für mindestens einen vierwertigen Rest der Formeln IV, V, VI oder VII

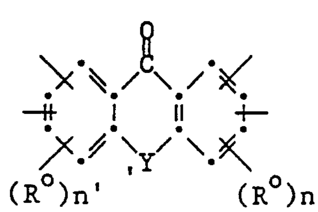 (IV), 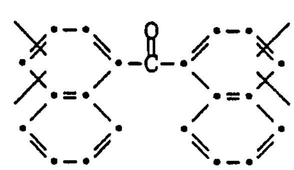 (V)

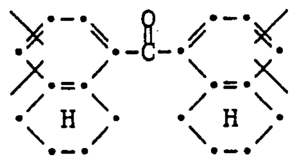 (VI) 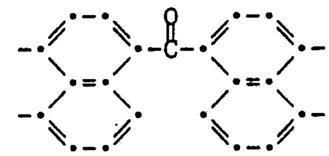 (VII)

steht, in denen die freien Bindungen in Orthostellung zueinander gebunden sind und Y eine direkte Bindung, $—CH_2—$, $—(CH_2)_2—$, —O—, —S—, —SO—, $—SO_2—$, —CO—, —NR— oder $—CRR^1—$ bedeutet, wobei R ein Wasserstoffatom, $C_1—C_{10}$-Alkyl, Phenyl, Naphthyl oder Phenyl$(C_aH_{2a})$— mit a = 1 bis 4 ist und $R^1$ die Bedeutung von R mit Ausnahme der Bedeutung eines Wasserstoffatomes hat, $R^0$ für $C_1—C_{10}$-Alkyl, Halogen, —CN, $—NO_2$, $C_1—C_{12}$-Alkoxy, Phenoxy, Naphthoxy oder Phenyl-$(C_a—H_{2a})$— mit a = 1—4 steht, n' = 0, 1 oder 2 ist,

X ein unsubtituierter oder substituierter zweiwertiger aliphatischer Rest, der durch Heteroatome, aromatische, heterocyclische oder cycloaliphatische Gruppen unterbrochen sein kann, ein unsubstituierter oder substituierter heterocyclischer, cycloaliphatischer oder araliphatischer Rest, ein aromatischer Rest, bei dem zwei Arylkerne über eine aliphatische Gruppe verknüpft sind, oder ein durch mindestens eine Alkylgruppe, Cycloalkylgruppe, Alkoxygruppe, Alkoxyalkylgruppe, Alkythiogruppe, Alkylthioalkylgruppe, Hydroxyalkylgruppe, Hydroxyalkoxygruppe, Hydroxyalkylthiogruppe, Aralkylgruppe, oder zwei benachbarte C-Atome des aromatischen Restes durch eine Alkylengruppe substituierter aromatischer Rest ist,

Q' ein dreiwertiger aromatischer Rest ist,

Z' die gleiche Bedeutung wie Z hat, oder ein von Z verschiedener vierwertiger organischer Rest ist, und

X' der zweiwertige, von X verschiedene Reste eines organischen Diamines ist, wobei Z in Formel I auch vierwertige Benzophenonreste darstellen kann, wenn Strukturelemente der Formel III enthalten sind, flächenmässig oder unter einer Photomaske bestrahlt und die unbelichteten Anteile anschliessend mit einem Lösungsmittel entfernt.

Die Strukturelemente der Formel I sind bevorzugt in einer Menge von 5—100 Mol-%, bevorzugter 30—100 Mol-%, besonders 60—100 Mol-%, und insbesondere 80—100 Mol-% und die Struckturelement der Formel II und/oder III bevorzugt in einer Menge von 95—0 Mol-%, bevorzugter 70 bis 0 Mol-%, besonders 60 bis 0 Mol-% und insbesondere 20 bis 0 Mol-% enthalten.

Die freien Bindungen in den Resten der Formeln IV bis VI sind bevorzugt in Meta- und Parastellung zur CO-Gruppe gebunden.

$R^0$ als Alkyl oder Alkoxy kann linear oder verzweigt sein un enthält vorzugsweise 1 bis 4 C-Atome. Beispiele sind Methyl, Methoxy, Ethyl, Ethoxy, n-Propyl, n-Propoxy, Isopropyl, Isoproxy, Butyl und Butoxy.

$R^0$ als Halogen ist bevorzugt Chlor und in der $—C_aH_{2a}$-Gruppe ist a bevorzugt 1 oder 2. In Formel IV steht n' bevorzugt für Null.

R bzw. $R^1$ als Alkyl enthalten bevorzugt 1 bis 4 C-Atome. Das Alkyl kann linear oder verzweigt sein. Beispiele sind Methyl, Ethyl, n-Propyl, Isopropyl und Butyl. In der $—(C_aH_{2a})$-Gruppe ist a bevorzugt 1 oder 2.

Y in Formel IV ist bevorzugt eine direkte Bindung, —O—, —S—, $—CH_2—$ oder —CO—.

Z' als von Z verschiedener Rest enthält als vierwertiger aromatischer Rest bevorzugt 6 bis 30, besonders 6 bis 20 C-Atome. In einer bevorzugten Untergruppe entspricht Z' den Formeln

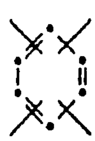 , 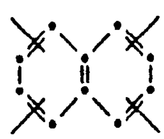 ,

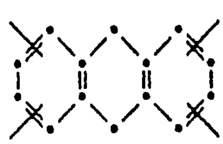 , 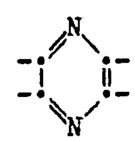 , 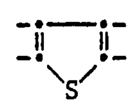

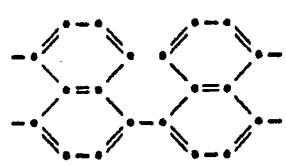 , 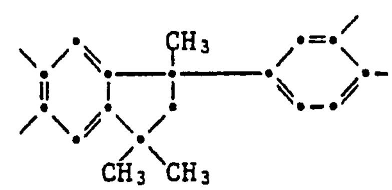

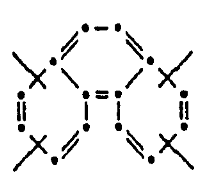 , 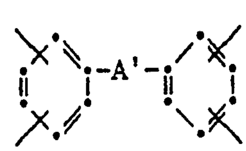

worin A' eine direkte Bindung oder eine Brückengruppe der Formeln

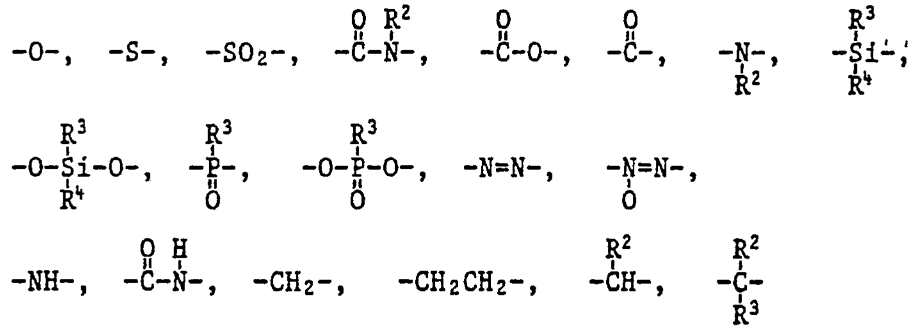

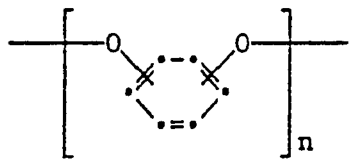

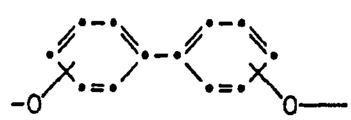

ist, worin $R^2$, $R^3$ und $R^4$ Alkyl mit 1 bis 6 C-Atomen, Phenyl oder Benzyl und $R^3$ und $R^4$ Alkoxy mit 1 bis 6 C-Atomen, Phenyloxy oder Benzyloxy sind und n für Null oder die Zahl 1, 2 oder 3 steht.

In den vorausstehenden Formeln befinden sich stets je zwei der freien Bindungen in Peri- und/oder Orthostellung.

Eine bevorzugte Untergruppe für Z' sind Reste der Formeln

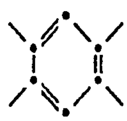

oder

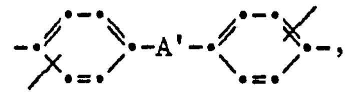

worin A' eine direkte Bindung, —O—, —$SO_2$—, —$CH_2$— und besonders —CO— ist.

Ganz besonders bevorzugt sind Reste der Formeln

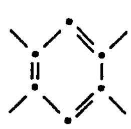

und besonders

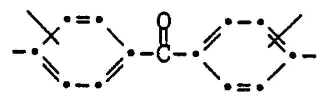

oder Mischungen davon, z.B. solche mit mindestens 5 Mol-% vierwertigen Benzophenonresten. Die freien Bindungen im Benzophenonrest befinden sich in Orthostellung. Es wurde gefunden, dass Benzophenonreste ebenfalls zu autophotovernetzbaren Polyimiden führen. Bei geringeren Gehalten an Struckturelementen der Formel I kann die Lichtempfindlichkeit durch einen Gehalt an Benzophenontetracarbonsäureimideinheiten gesteigert werden.

Beispiele für Tetracarbonsäureanhydride mit einem Rest Z' sind:
2,3,9,10-Perylentetracarbonsäuredianhydrid,
1,4,5,8-Naphthalintetracarbonsäuredianhydrid,
2,6-Dichlornaphthalin-1,4,5,8-tetracarbonsäuredianhydrid,
2,7-Dichlornaphthalin-1,4,5,8-tetracarbonsäuredianhydrid,
2,3,6,7-Tetrachlornaphthalin-1,4,5,8-tetracarbonsäuredianhydrid,
Phenanthren-1,8,9,10-tetracarbonsäuredianhydrid,
Pyromellitsäuredianhydrid,
3,3',4,4'-Biphenyltetracarbonsäuredianhydrid,
2,2',3,3'-Biphenyltetracarbonsäuredianhydrid,
4,4'-Isopropylidendiphthalsäureanhydrid,
3,3'-Isopropylidendiphthalsäureanhydrid,
4,4'-Oxydiphthalsäureanhydrid,
4,4'-Sulfonyldiphthalsäureanhydrid,
3,3'-Oxydiphthalsäureanhydrid,
4,4'-Methylendiphthalsäureanhydrid,
4,4'-Thiodiphthalsäureanhydrid,
4,4'-Aethylidendiphthalsäureanhydrid,
2,3,6,7-Naphthalintetracarbonsäuredianhydrid,
1,2,4,5-Naphthalintetracarbonsäuredianhydrid,
1,2,5,6-Naphthalintetracarbonsäuredianhydrid,
Benzol-1,2,3,4-tetracarbonsäuredianhydrid,

Thiophen-2,3,4-5-tetracarbonsäuredianhydrid,
1-(3',4'-Dicarboxyphenyl)-1,3,3-trimethylindan-5,6-dicarbonsäuredianhydrid,
1-(3',4'-Dicarboxyphenyl)-1,3,3-trimethylindan-6,7-dicarbonsäuredianhydrid,
1-(3',4'-Dicarboxyphenyl)-3-methylindan-5,6-dicarbonsäuredianhydrid,
1-(3',4'-Dicarboxyphenyl)-3-methylindan-6,7-dicarbonsäuredianhydrid,
3,3',4,4'-Benzophenontetracarbonsäureanhydrid,
4,5,3',4'-Benzophenontetracarbonsäureanhydrid.

Q' in Formel III ist der dreiwertige aromatische rest einer Aminodicarbonsäure. Bei den aromatischen Resten handelt es sich vorzugsweise um ein- oder zweikernige Phenylreste, wie z.B. Phenyl, Naphthyl oder Bisphenylene. Die Reste können durch Halogen, z.B. Chlor, oder Cyano oder Nitro substituiert sein. In einer bevorzugten Ausführungsform Q' in Formel III einenen dreiwertigen Rest der Formeln

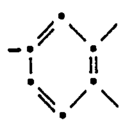 , 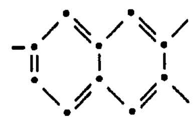 oder 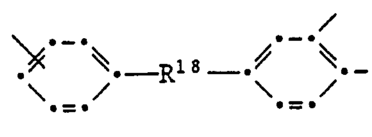

worin die freie Bindung in Meta- oder Parastellung zur $R^{18}$-Gruppe gebunden ist und $R^{18}$ eine direkte Bindung oder eine Brückengruppe ist. Geeignete Brückengruppen sind z.B. $—CH_2—$, —O—, —S— und besonders —CO—. Besonders bevorzugt entspricht der dreiwertige Rest der Formel

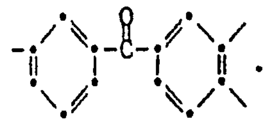 .

Wenn die Polyimide einen Rest der Formel III enthalten, kann Z in Formel I auch nur der vierwertige Rest einer Benzophenontetracarbonsäure sein, z.B. solche der Formeln,

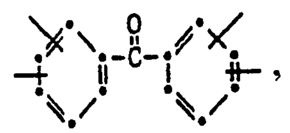 ,

worin die freien Bindungen in Orthostellung gebunden sind.

X im Strukturelement der Formel enthält als zweiwertiger aliphatischer Rest bevorzugt 2 bis 30 C-Atome, als heterocyclischer aliphatischer Rest bevorzugt 3 bis 5 Ring-C-Atome und 1 bis 2 Heteroatome, z.B. N, O oder S, als cycloaliphatischer Rest 5 bis 8 Ring-C-Atome, als araliphatischer Rest 7 bis 30 C-Atome, als aromatischer Rest mit verknüpften Arylkernen 13 bis 30 C-Atome und als substituierter aromatischer Rest 7 bis 30 C-Atome. Bevorzugter ist X ein aliphatischer Rest mit 3 bis 30 C-Atomen, ein cycloaliphatischer Rest mit 5-8 Ring-C-Atomen, ein araliphatischer Rest mit 7 bis 30 C-Atomen oder ein substituierter aromatischer Rest mit 7 bis 30 C-Atomen.

X in Formel I in der Bedeutung als zweiwertiger aliphatischer Rest enthält bevorzugt 6 bis 30 und insbesondere 6 bis 20 C-Atome. In einer bevorzugten Untergruppe ist X lineares oder verzweigtes Alkylen, das durch Sauerstoffatome, —NH—, $—NR^a—$, $—^{\oplus}NR_2^aG^{\ominus}—$, Cyclohexylen, Naphthylen, Phenylen oder Hydantoinreste unterbrochen sein kann. $R^a$ kann z.B. Alkyl mit 1 bis 12-C-Atomen oder Cycloalkyl mit 5 oder 6 Ring-C-Atomen, Phenyl oder Benzyl sein. $G^{\ominus}$ bedeutet ein Anion einer Protonensäure, z.B. Halogenid, Sulfat, Phosphat. In einer bevorzugten Ausführungsform ist X lineares oder verzweigtes Alkylen mit 6 bis 30 C-Atomen, $—(CH_2)_m—R^{13}—(CH_2)_n—$, worin $R^{13}$ Phenylen, Naphthylen, Cyclopentylen oder Cyclohexylen und m und n unabhängig voneinander die Zahl 1, 2 oder 3 sind, $—R^{14}(OR^{15})O—R^{14}—$, worin $R^{14}$ Aethylen, 1,2-Propylen, 1,3-Propylen oder 2-Methyl-1,3-propylen und $R^{15}$ Aethylen, 1,2-Propylen, 1,2-Butylen, 1,3-Propylen oder 1,4-Butylen und p eine Zahl von 1 bis 100 sind, oder

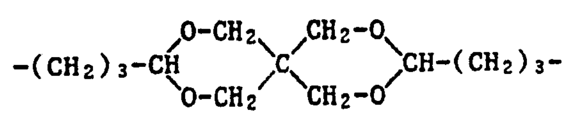

Beispiele für aliphatische Reste sind: Methylen, Aethylen, 1,2- oder 1,3-Propylen, 2,2-Dimethyl-1,3-propylen, 1,2-, 1,3- oder 1,4-Butylen, 1,3- oder 1,5-Pentylen, Hexylene, Heptylene, Octylene, Decylene, Dodecylene, Tetradecylene, Hexadecylene, Octadecylene, Eicosylene, 2,4,4-Trimethylhexylen, 1,10-Dialkyldecylene, wobei das Alkyl bevorzugt 1 bis 6 C-Atome aufweist, substituierte, 1,11-Undecylene, wie sei z.B. in der EP—B—0 011 559 beschrieben sind, Reste von Jeffaminen wie z.B.

$—(CH_2)_3(OCHCH_2)_pO(CH_2)_3$ mit p = 1 bis 100 oder
$CH_3$ (an CH)

$-(CH_2)_3-(O(CH_2)_4)_p-O-(CH_2)_3-$ mit p = 1—100, Dimethylencyclohexan, Xylylen und Diäthylenbenzol.

Bei den durch heterocyclische Reste unterbrochenen aliphatischen Resten kann es sich z.B. um solche handeln, die sich von N,N'-aminoalkylierten Hydantoinen oder Benzimidazolen ableiten. Beispiele sind N,N'-(γ-Aminopropyl)-5,5-dimethyl-hydantoin oder -benzimidazolon und solche der formel

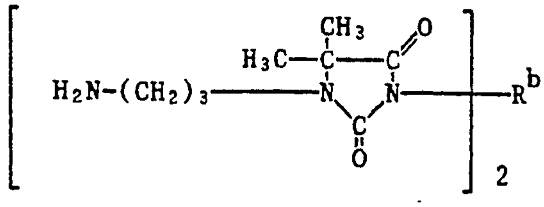

worin $R^b$ Alkylen mit 1 bis 12, bevorzugt 1 bis 4 C-Atomen oder

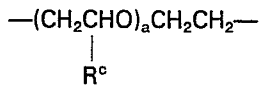

ist, worin $R^c$ in Wasserstoffatom oder Methyl und a ganze Zahlen von 1—20 bedeuten.

X kann als zweiwertiger aliphatischer Rest aush ein Siloxangruppen enthaltender zweiwertiger Rest sein. Dieser kann der Formel

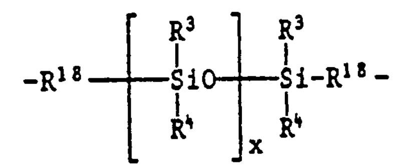

entsprechen, worin x für eine rationale Zahl von mindestens 1 steht, $R^3$ und $R^4$ die zuvor angegebene Bedeutung haben und $R^{18}$ ein zweiwertiger Kohlenwasserstoffrest ist, z.B. Alkylen mit 1 bis 12, vorzugsweise 1 bis 6 C-Atomen, Cycloalkylen mit bevorzugt 5 oder 6 Ringkohlenstoffatomen oder Phenylen. $R^3$ und $R^4$ sind bevorzugt Methyl oder Phenyl und x bevorzugt eine Zahl von 1 bis 1000, besonders 1 bis 100 und insbesondere 1—10. Beispiele für Alkylen sind Aethylen, 1,3- oder 1,2-Propylen, 1,3- oder 1,4-Butylen. Diamine mit dieser Gruppe X sind in der US—PS 4 030 948 beschrieben. Weitere geeignete Diamine mit einer Siloxangruppen enthaltenden Gruppe X sind in der US—PS 3 435 002 und EP—A—0 054 426 beschrieben.

Geeignete Substituenten für die aliphatischen Reste sind z.B. Hydroxyl, Halogenid wie F oder Cl oder Alkoxy mit 1 bis 6 C-Atomen.

X in Formel I in seiner Bedeutung als zweiwertiger cycloaliphatischer Rest enthält bevorzugt 5 bis 8 Ring-C-Atome und ist besonders unsubstituiertes, oder mit Alkyl, das vorzugsweise 1 bis 4 C-Atome enthält, substituiertes ein- oder zweikerniges Cycloalkylen mit 5 bis 7 Ring-C-Atomen. In einer bevorzugten Ausführungsform ist X als cycloaliphatischer Rest ein solcher der Formeln

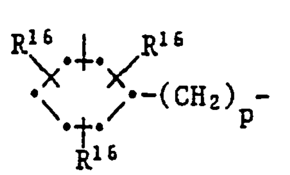 oder 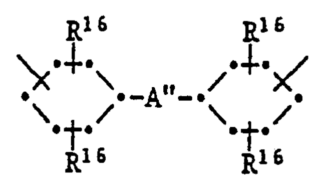,

worin p Null oder die Zahl 1 ist, die $R^{16}$ unabhängig Wasserstoff oder Alkyl mit 1 bis 6 C-Atomen bedeutet und A'' für eine direkte Bindung, —O—, —S—, $-SO_2-$, Alkylen mit 1 bis 3 C-Atomen oder Alkyliden mit 2 bis 6 C-Atomen steht. $R^{16}$ ist bevorzugt Aethyl und Methyl, A'' bevorzugt Methylen, und das Alkyliden enthält bevorzugt 2 oder 3 C-Atome, wie Aethyliden und 1,1- oder 2,2-Propyliden.

Beispiele für X als Cycloalkylen sind: 1,2- oder 1,3-Cyclopentylen, 1,2-, 1,3-oder 1,4-Cyclohexylen, Cycloheptylen, Cyclooctylen, Methylcyclopentylen, Methyl- oder Dimethylencyclohexylen, 3- oder 4-Methylencyclohex-1-yl, 5-Methyl-3-methylencyclohexy-1-yl, 3,3'-oder 4,4'-Bis-cyclohexylen, 3,3'-Dimethyl-4,4'-biscyclohexylen, 4,4'-Biscyclohexylenäther der -sulfon oder -methan oder -2,2-propan, sowie die Reste von Bis-aminomethyltricyclodecan, Bis-aminomethyltricyclodecan, Bis-aminomethylnorbornan und Menthandiamin.

Besonders bevorzugt ist X als cycloaliphatischer Rest 1,4- oder 1,3-Cyclohexylen, 2,2,6-Trimethyl-6-methylen-cyclohex-4-yl, Methylenbis(cylohex-4-yl) oder Methylenbis(3-methylcyclohex-4-yl).

Heterocyclische Diaminreste X leiten sich bevorzugt von N-heterocyclischen Diaminen ab, z.B. von Pyrrolidin, Indol, Piperidin, Pyridin, Pyrrol, deren N-Atom alkyliert, z.B. methyliert sein kann. Ein Beispiel ist N-Methyl-4-amino-5-aminomethylpiperidin.

X als araliphatischer Rest enthält bevorzugt 7 bis 30 C-Atome. Die aromatischen Gruppen sind bevorzugt so substituiert wie X als aromatischer Rest, einschliesslich der Bevorzugungen, zumindest aber monosubstituiert, bevorzugt in Orthostellung zum N-Atom. Der araliphatische Rest enthält besonders 8 bis 26 C-Atome. Der aromatische Rest im araliphatischen Rest ist bevorzugt ein Phenylrest. In seiner Bedeutung als araliphatischer Rest ist X besonders unsubstituiertes oder am Aryl durch Alkyl substituiertes Aralkylen, wobei der Alkylenrest linear oder verzweigt ist. In einer bevorzugten Aufführungsform entspricht der araliphatische Rest der Formel

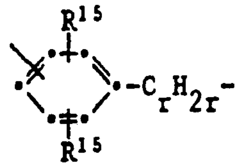

worin die $R^{15}$ unabhängig voneinander ein Wasserstoffatom oder besonders Alkyl mit 1—6 C-Atomen und r ganze Zahlen von 1—20 bedeuten.

Die freie Bindung befindet sich bevorzugt in m-Stellung oder p-Stellung zur $C_rH_{2r}$-Gruppe und eine oder beide $R^{15}$ sind bevorzugt in o-Stellung zur freien Bindung gebunden.

Beispiele für X als araliphatischer Rest sind: m- oder p-Benzylen, 3-Methyl-p-benzylen, 3-Aethyl-p-Benzylen, 3,5-Dimethyl-p-benzylen, 3,5-Diäthyl-p-benzylen, 3-Methyl-5-äthyl-p-benzylen, p-Phenylen-propylen, 3-Methyl-p-phenylen-propylen, p-Phenylbutylen, 3-Aethyl-p-phenylenpentylen sowie insbesondere längerkettige Phenylenalkylenreste, die z.B. in der EP—A—0 069 062 beschrieben sind: 6-(p-Phenylen)-6-methylhept-2-yl, 6-(3′-Methyl-p-phenylen)-6-methylhept-2-yl, 6-(3′-Aethyl-p-phenylen)-6-methylhept-2-yl, 6-(3′,5′-Dimethyl-p-phenylen)-6-methylhept-2-yl, 11-(-p-Phenylen)-2,11-dimethyl-dodecyl-1-yl, 13-(-p-Phenylen)-1,12-dimethyltetradec-3-yl.

X kann auch ein aromatischer Rest sein, bei dem zwei Arylkerne, besonders Phenyl über eine aliphatische Gruppe verknüpft sind. Dieser Rest entspricht bevorzugt der Formel

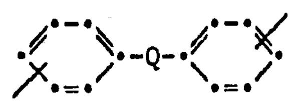

worin die freien Bindungen in p-, m- und besonders o-Stellung zur Q-Gruppe gebunden sind un Q $C_2$—$C_6$-Alkyliden, $C_1$—$C_{12}$-, besonders $C_1$—$C_6$-Alkylen ist, das durch O oder S unterbrochen sein kann. Beispiele für Q sind Aethylen, 1,2- oder 1,3-Propylen, Butylen, $—CH_2—O—CH_2$, $—CH_2—S—CH_2—$ und $—CH_2CH_2—O—CH_2CH_2—$. Die Phenylreste können durch Gruppen wie Fluor, Chlor, Carboxy und deren Ester substituiert sein.

Beispiele sind 4,4′-Methylenbis-(o-chloranilin),5,5′-Methylenbis(aminophenol), 4,4-Methylendianilin, 4,4′-Aethylidendianilin, 4,4′-Propylidendianilin, 4,4′-Methylenbis(3-carboxyanilin) und deren Ester.

Unter den Homo- und Copolyimiden sind besonders solche mit Strukturelementen der Formel I bevorzugt, in denen X substituierte aromatische Reste bedeutet. Der Substituent am aromatischen Rest enthält bevorzugt 1 bis 20, besonders 1—12 und insbesondere 1—6 C-Atome. Insbesondere ist der Substituent Cycloalkyl mit 5 oder 6 Ringkohlenstoffatomen, lineares oder verzweigtes Alkyl, Alkoxy, Alkylthio, Hydroxyalkyl, Hydroxyalkoxy oder Hydroxyalkylthio mit 1 bis 12, besonders 1—6 C-Atomen, Alkoxyalkyl, Alkylthioalkyl mit 2 bis 12, besonders 2—6 C-Atomen, Benzyl, Trimethylen oder Tetramethylen. Als Alkoxyalkyl ist Alkoxymethyl bevorzugt und als Alkoxy Methoxy. Beispiele für den Substituenten sind: Methyl, Ethyl, Propyl, Isopropyl, n-Butyl, Isobutyl, Pentyl, Hexyl, Octyl, Dodecyl, Tetradecyl, Eicosyl, Methoxy, Ethoxy, Propoxy, Butoxy, Pentoxy, Hexoxy, Methoxymethyl, Methoxyethyl, Aethoxymethyl, Propoxymethyl, Butoxymethyl, Benzyl, Methylbenzyl, Phenylethyl, Methylthio, Aethylthio, Hydroxyethyl, Methylthioethyl und Hydroxyethylthio. Bevorzugte Reste sind Methoxymethyl, Ethoxymethyl, Methyl, Ethyl, n-Propyl, i-Propyl, Trimethylen und Tetramethylen, Cyclopentyl und Cyclohexyl. Besonders bevorzugt ist insbesondere Methyl sowie Ethyl und i-Propyl. Bei dem substituierten aromatischen Rest kann es sich um einkernige oder mehrkernige, besonders zweikernige Reste handeln, insbesondere um ein- oder zweikernige Phenylenreste. Einkernige Reste können bis zu 4, bevorzugt 2 Substituenten enthalten und zweikernige Reste können bis zu 4, bevorzugt 1 oder 2 Substituenten in jedem Kern enthalten. Es wurde gefunden, dass die Lichtempfindlichkeit von jenen Homo- oder Copolyimiden besonders hoch ist, bei denen ein oder zwei Substituenten in Orthostellung zum N-Atom gebunden sind. Die Substitution in Orthostellung ist daher bevorzugt. Der aromatische Rest ist bevorzugt in Meta- und Parastellung zum N-Atom gebunden. In einer bevorzugten Untergruppe enthält der Substituent das aromatischen Restes als Alkyl oder Alkoxy 1 bis 20 C-Atome, als Alkoxyalkyl 2 bis 12 C-Atome, als Cycloalkyl 5 oder 6 Ringkohlenstoffatome und als Alkylen 3 oder 4 C-Atome und als Aralkyl Benzyl. Der Substituent ist bevorzugt Alkyl mit 1 bis 4 C-Atomen, besonders Isopropyl, Ethyl und besonders Methyl.

X kann als substituierter aromatischer Rest 7 bis 30, besonders 8 bis 25 C-Atome enthalten. Der aromatische Rest ist bevorzugt ein Pyridinrest und besonders ein Kohlenwasserstoffrest, der durch Alkyl, Alkoxyalkyl, Alkoxy, Trimethylen oder Tetramethylen substituiert ist. Der aromatische Rest kann weiter

Substituenten enthalten, z.B. Halogenid wie Cl oder Br. In einer bevorzugten Untergruppe sind die aromatischen Reste als einkernige Reste Phenylreste und als zweikernige Reste Naphthylen oder Bisphenylenreste.

Eine bevorzugte Untergruppe von erfindungsgemäss zu verwendenden Polyimiden sind solche, in denen X als substituierter aromatischer Rest den Formeln VIII, VIIIa und/oder VIIIb entspricht,

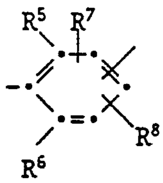 (VIII)

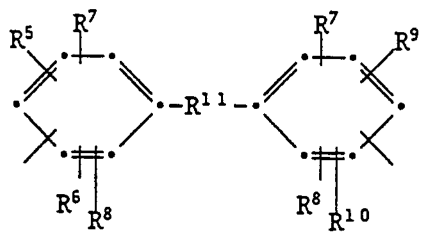 (VIIIa)

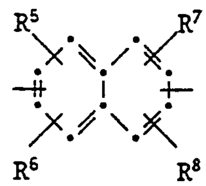 (VIIIb),

worin in Formel VIII die freien Bindungen in Meta- oder Parastellung zueinander stehen, in Formel VIIIa die freien Bindungen bevorzugt in Meta- oder Parastellung zur $R^{11}$4-Gruppe stehen und $R^5$ und $R^6$ in den beiden Orthostellungen der freien Bindung gebunden sind, und in Formel VIIIb die freien Bindungen in 2-, 3-, 6- und 7-Stellung gebunden sind un $R^5$ und $R^6$ sich in den beiden Orthostellugen der freien Bindungen befinden, $R^{11}$ für eine direkte Bindung, —O—, —S—, —SS—, —SO—, —$SO_2$—, —CO—, —COO—, —NH—,

—N—Alkyl

mit 1 bis 6 C-Atomen im Alkyl,

—N-Phenyl,

—N-Benzyl,

—CONH—, —CON-Alkyl- mit 1 bis 6 C-Atomen im Alkyl, —CON-Phenyl-, —CON-Benzyl-,

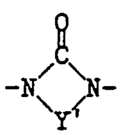 , worin Y' für 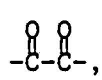, 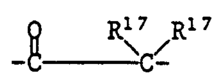 oder 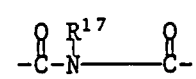

steht und $R^{17}$ ein Wasserstoffatom, $C_1$—$C_6$-Alkyl oder Phenyl bedeutet, lineares oder verzweigtes Alkylen mit 1 bis 3 C-Atomen, gegebenenfalls durch Cl oder F substituiertes Alkyliden mit 2 bis 12 C-Atomen, Cycloalkyliden mit 5 oder 6 Ringkohlenstoffatomen, Phenylen, Phenylendioxy, oder die Gruppe

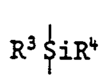, 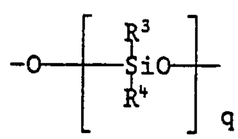 oder

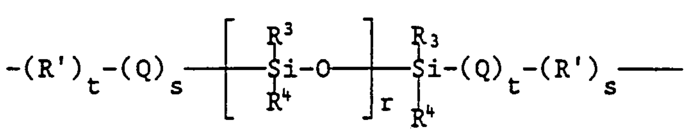 steht, worin $R^3$

und $R^4$ Alkyl oder Alkoxy mit 1 bis 6 C-Atomen, Phenyl, Benzyl, Phenyloxy oder Benzyloxy sind, r eine Zahl 1 von 1 bis 10, t Null oder die Zahl 1 und s Null oder die Zahl 1 ist, und R'—O— oder —S— und Q $C_1$—$C_6$-

Xanthontetracarbonsäure erhält man, indem man zunächst 3,3',4,4'-Tetramethylphenyläther mit $CCl_4$

Atomen, Alkoxyalkyl mit 2 bis 12 C-Atomen, Cyclopentyl, Cyclohexyl oder Benzyl sind oder in den Formeln VIII oder VIIIa $R^5$ und $R^7$ in Nachbarstellung gebunden sind und zusammen Trimethylen oder Tetramethylen bedeuten, wobei $R^6$ auch ein Wasserstoffatom sein kann, $R^7$ und $R^8$ ein Wasserstoffatom sind oder unabhängig die Bedeutung von $R^5$ und $R^6$ haben, und, $R^9$ und $R^{10}$ ein Wasserstoffatom sind, unabhängig die Bedeutung von $R^5$ und $R^6$ haben oder $R^7$ und $R^9$ in Formel VIIIa zusammen Trimethylen oder Tetramethylen sind. $R^5$ und $R^6$ sind bevorzugt Alkyl mit 1 bis 6 C-Atomen, besonders Methyl, Aethyl, n-Propyl und Isopropyl. Die freien Bedinungen der Formel VIIIa befinden sich bevorzugt in Meta-und besonders Parastellung zur $R^{11}$-Gruppe. Das Alkyl in den $R^{11}$-Resten kann z.B. Methyl, Aethyl, Propyl, Isopropyl, n-Butyl oder Pentyl sein. $R^{11}$ als Alkylen ist bevorzugt Aethylen und besonders Methylen. Als Alkyliden enthält $R^{11}$ bevorzugt 2 bis 6 C-Atome. Beispiele sind Aethyliden, 2,2-Butyliden, 2,2- oder 3,3-Pentyliden, Hexafluorpropyliden und besonders 2,2-Propyliden. $R^{11}$ als Cycloalkyliden kann z.B. Cyclopentyliden und besonders Cyclohexyliden sein. Die $R^{11}$-Gruppe ist bevorzugt eine direkte Bindung, —O—, —S—, —$SO_2$—, —CO—, Alkylen und Alkyliden. Besonders bevorzugt ist $R^{11}$ eine direkte Bindung, —O—, und insbesondere —CO-oder —$CH_2$—. $R^3$ und $R^4$ sind bevorzugt Alkyl, besonders Methyl oder Phenyl. R' steht bevorzugt für —O— und Q bevorzugt für Methylen oder Aethylen, q ist bevorzugt eine Zahl von 1 bis 10 und r eine Zahl von 1—20, besonders 1—10.

Eine weitere bevorzugte Gruppe von substituierten aromatischen Diaminresten sind solche der Formel

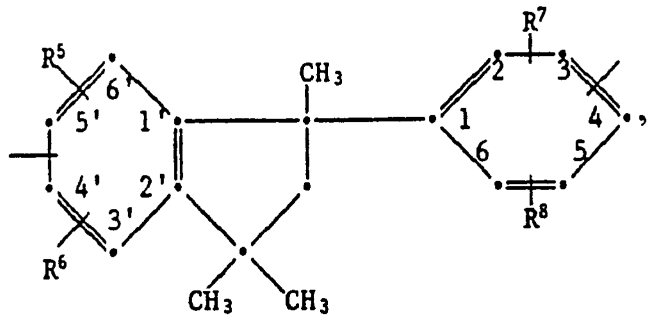

worin die freie Bindung in 4'- oder 5'-Stellung gebunden ist und die andere in 3-, 5-, und bevorzugt 4-Stellung, $R^5$ und $R^6$ und/oder $R^7$ und $R^8$ sich in den Orthostellungen der freien Bindung befinden und für Alkyl oder Alkoxy mit 1 bis 12 C-Atomen oder Alkoxyalkyl mit 2 bis 12 C-Atomen stehen.

Eine besonders bevorzugte Untergruppe von erfindungsgemässen Polyimiden sind jene, in denen X in Formel I Resten der Formeln

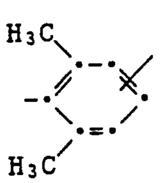 , 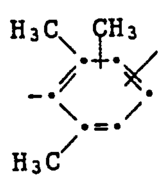 , 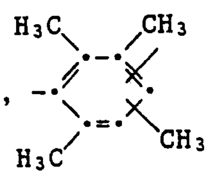 , 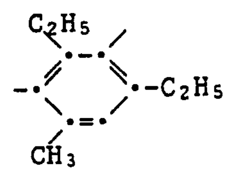

entspricht, worin die freien Bindungen in Meta- oder Parastellung zueinander stehen, oder der Formel

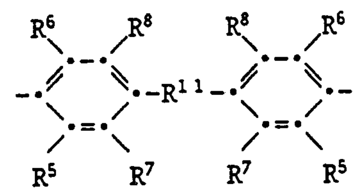

entsprechen, worin $R^5$ und $R^6$ unabhängig Methyl, Aethyl, n-Propyl oder Isopropyl und $R^7$ und $R^8$ ein Wasserstoffatom sind oder die Bedeutung von $R^5$ haben oder $R^5$ und $R^7$ zusammen Tri- oder Tetramethylen bedeuten und $R^6$ und $R^8$ ein Wasserstoffatom sind, und $R^{11}$ eine direkte Bindung, $CH_2$, 2,2-Propyliden oder CO ist. Unter diesen zweikernigen Resten sind besonders solche bevorzugt, in denen $R^5$, $R^6$, $R^7$ und $R^8$ für Methyl stehen. Copolyimide, die mindestens 2 verschiedene Reste dieser Formeln enthalten, sind eine weitere bevorzugte Ausführungsform der Erfindung.

Die erfindungsgemäss zu verwendenden Copolyimide enthalten mindestens zwei verschiedene Strukturelemente, wobei sich die Anzahl an verschidenen Strukturelementen im wesentlichen nach den gewünschten Eigenschaften und dem Anwendungsgebiet richten. Bevorzugt enthalten sie 2 bis 4 verschiedene Strukturelemente, wobei sich die Strucktureiemente nur im Rest X der Formel I unterscheiden können. In einer besonders bevorzugten Ausführungsform solcher Copolyimide sind Strukturelemente von Ortho-disubstituierten Phenylenen enthalten, besonders von 1,3-Phenylenen.

Beispiele für X sind: 2-Methyl-1,4-phenylen, 2,6-Dimethyl-1,4- oder -1,3-phenylen, 2,6-Diäthyl-1,4- oder

-1,3-phenylen, 2,6-Dimethyl-5-chlor-1,4- oder -1,3-phenylen, 2-Methyl-6-äthyl-1,4- oder -1,3-phenylen, 2-Methyl-6-isopropyl-1,4-oder -1,3-phenylen, 2,6-Diisopropyl-1,4- oder -1,3-phenylen, 2,6-Dimethoxy-1,4-oder -1,3-phenylen, 2,6-Diäthoxy-1,4- oder -1,3-phenylen, 2-Methyl-6-methoxy-1,4-oder -1,3-phenylen, 2,6-Dibenzyl-1,4- oder -1,3-phenylen, 2,6-Dimethoxymethyl-1,4- oder -1,3-phenylen, 2,5,6-Trimethyl-1,4-oder 1,3-phenylen, 2,5,6-Triäthyl-1,4- oder -1,3-phenylen, 2,4,6-Trimethyl-1,3-phenylen, 2,3,5,6-Tetramethyl-1,4-phenylen, 2,4,5,6-Tetramethyl-1,3-phenylen, Tetrahydro-1,4-oder -1,4-naphthylen, Reste der Formeln

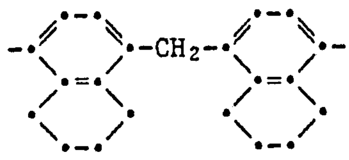
,
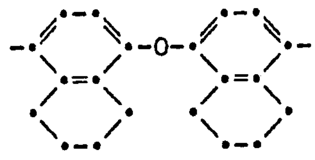
,

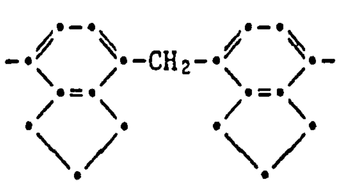
,
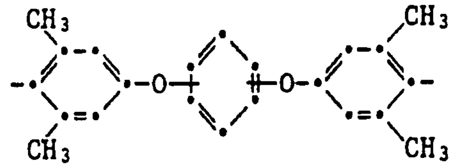

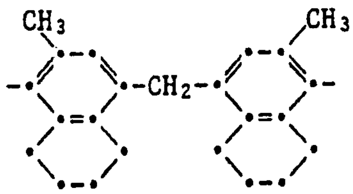
,
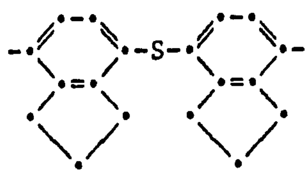
,

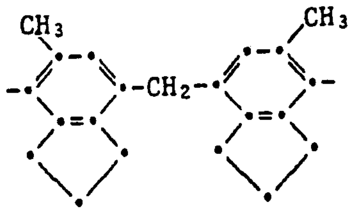
,
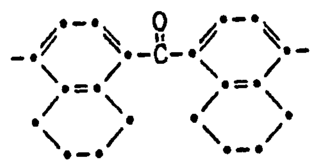
,

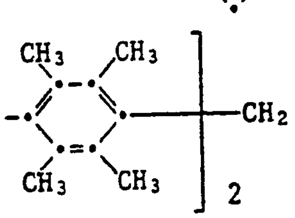
,
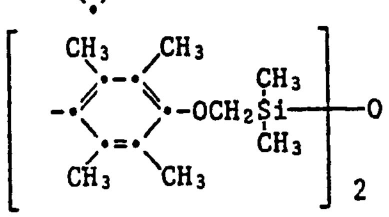
,
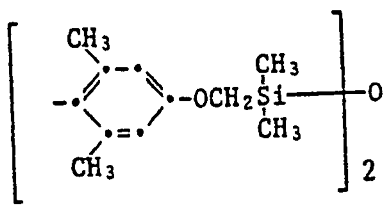
,

sowie

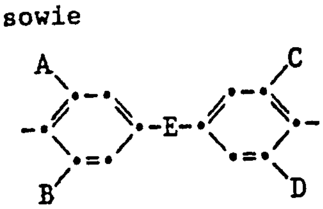
,
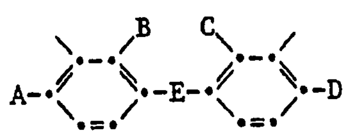

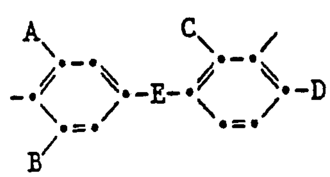
,
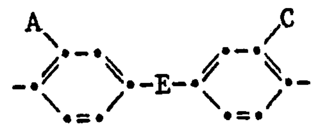
,

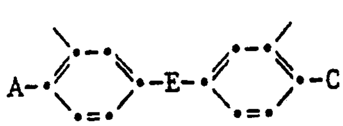
und
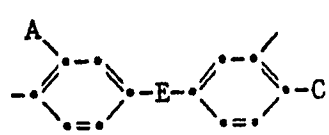

worin A, B, C, D und E die in nachfolgender Tabelle angegebenen Bedeutungen haben. Die freien Positionen in den Phenylkernen können hierbei durch einen oder zwei weitere Substituenten G oder H in jedes Kern besetz sein, wobei G oder H die in nachfolgender Tabelle für A angegebene Bedeutung haben können:

| E | A | B | C | D |
|---|---|---|---|---|
| $CH_2$ | Methyl | Methyl | H | H |
| $CH_2$ | Methyl | Aethyl | H | H |
| $CH_2$ | Aethyl | Aethyl | H | H |
| $CH_2$ | Isopropyl | Isopropyl | H | H |
| $CH_2$ | Methoxymethyl | | H | H |
| $CH_2$ | Benzyl | Benzyl | H | H |
| $CH_2$ | Methyl | Methyl | Methyl | H |
| $CH_2$ | Aethyl | Aethyl | Aethyl | H |
| $CH_2$ | Isopropyl | Isopropyl | Methyl | Methyl |
| $CH_2$ | Methoxymethyl | | Methyl | H |
| $CH_2$ | Methyl | Aethyl | Methyl | H |
| $CH_2$ | Methoxymethyl | | Methoxymethyl | |
| $CH_2$ | Methyl | Methyl | Methyl | Methyl |
| $CH_2$ | Aethyl | Aethyl | Aethyl | Aethyl |
| $CH_2$ | Methyl | Methyl | Aethyl | Aethyl |
| $CH_2$ | Aethyl | Aethyl | Isopropyl | Isopropyl |
| $CH_2$ | Isopropyl | Isopropyl | Isopropyl | Isopropyl |
| $CH_2$ | Isopropyl | Isopropyl | Methyl | H |
| $CH_2$ | Methoxy | Methoxy | Methyl | Methyl |
| O | Methyl | Methyl | H | H |
| O | Aethyl | Aethyl | H | H |
| O | Methyl | Methyl | Methyl | H |
| O | Methyl | Methyl | Methyl | Methyl |
| O | Methyl | Methyl | Aethyl | Aethyl |
| S | Methyl | Methyl | H | H |

| E | A | B | C | D |
|---|---|---|---|---|
| S | Aethyl | Aethyl | H | H |
| S | Methyl | Methyl | H | H |
| S | Methyl | Methyl | Methyl | Methyl |
| S | Aethyl | Aethyl | Aethyl | Aethyl |
| S | Methyl | Methyl | Aethyl | Aethyl |
| CO | Methyl | Methyl | Methyl | H |
| CO | Methyl | Methyl | H | H |
| CO | Methyl | Methyl | Methyl | Methyl |
| $SO_2$ | Methyl | Methyl | Aethyl | H |
| $SO_2$ | Methyl | Methyl | H | H |
| $SO_2$ | Methyl | Methyl | Methyl | Methyl |
| $SO_2$ | Aethyl | Aethyl | Methyl | Methyl |
| SO | Methyl | Methyl | Methyl | Methyl |
| SO | Methyl | Methyl | H | H |
| COO | Methyl | Methyl | H | H |
| COO | Methyl | Methyl | Methyl | Methyl |
| $CONCH_3$ | Methyl | Methyl | H | H |
| $NCH_3$ | Methyl | Methyl | Aethyl | Aethyl |
| $NCH_3$ | Methyl | Methyl | Methyl | Methyl |
| CONH | Methyl | Methyl | - | - |
| NH | Aethyl | Methyl | Aethyl | Methyl |
| NH | Methyl | Methyl | Methyl | Methyl |

| E | A | B | C | D |
|---|---|---|---|---|
| Si(Methyl)$_2$ | Methyl | Methyl | H | H |
| Si(Phenyl)$_2$ | Methyl | Methyl | Methyl | Methyl |
| Si(OMethyl)$_2$ | Aethyl | Aethyl | H | H |
| Si(OPhenyl)$_2$ | Methyl | Methyl | Methyl | Methyl |
| -OSi(Methyl)$_2$O- | Methyl | Methyl | Methyl | Methyl |
| Aethylen | Methyl | Methyl | H | H |
| Aethylen | Methyl | Methyl | Methyl | Methyl |
| Aethylen | Aethyl | Aethyl | H | H |
| Aethylen | Methyl | Methyl | Aethyl | Aethyl |
| Phenylen | Methyl | Methyl | Methyl | Methyl |
| Phenylen | Aethyl | Aethyl | H | H |
| $(CH_3)_2C=$ | Methyl | Aethyl | Methyl | Aethyl |
| $(CH_3)_2C=$ | Methyl | Methyl | Methyl | Methyl |
| $(CF_3)_2C=$ | Methyl | Methyl | Methyl | Methyl |
| direkte Bindung | Methyl | Methyl | H | H |
| direkte Bindung | Methyl | Aethyl | Methyl | Aethyl |
| direkte Bindung | Methyl | Aethyl | Methyl | H |
| direkte Bindung | Aethyl | Aethyl | Aethyl | Aethyl |
| direkte Bindung | Methoxy | Methoxy | Methoxy | Methoxy |
| direkte Bindung | Isopropyl | Isopropyl | H | H |
| direkte Bindung | Methoxy-methyl | Methoxy-methyl | Methoxy-methyl | Methoxy-methyl |

In einer weiteren bevorzugten Ausführungsform entspricht X als substituierter aromatischer Rest der Formel IX,

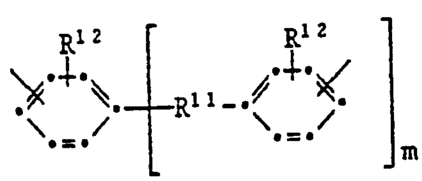

(IX),

worin m Null oder die Zahl 1 ist, die freien Bindungen in Meta- und bevorzugt in Orthostellung zur $R^{12}$-Gruppe gebunden sind, $R^{11}$ die gleiche Bedeutung wie in Formel VIIIa hat und $R^{12}$ die gleiche Bedeutung

wie $R^5$ hat. Die freien Bindungen befinden sich bevorzugt in Para- und besonders Metastellung zur $R^{11}$-Gruppe.

Eine bevorzugte Untergruppe sind Arylenreste der Formeln IXa, oder IXb oder IXc

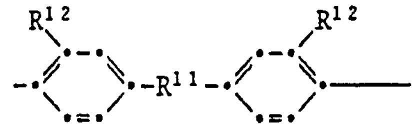 (IXa), 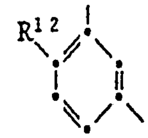 (IXb),

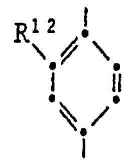 (IXc),

worin $R^{11}$ für eine direkte Bindung, —O—, —CO— oder —$CH_2$— steht und $R^{12}$ Methy, Aethyl, Isopropyl, Methoxy, Aethoxy oder ein Wasserstoffatom bedeutet.

Beispiele für Diamine $H_2N—X—NH_2$ mit einem aromatischen Rest der Formeln IX sind: 4,4'-Methylenbis-(3-methylanilin), 4,4'-Methylenbis-(2-äthylanilin), 4,4'-Methylenbis-(2-methoxyaniiin), 5,5'-Methylenbis-(2-aminophenol), 4,4'-Methylenbis-(2-methylanilin), 4,4'-Oxybis-(2-methoxyanilin), 4,4'-Thiobis-(2-methylanilin), 4,4'-Thiobis-(2-methoxyanilin), 4,4'-Sulfonylbis-(2-methylanilin), 4,4'-Sulfonylbis-(2-äthoxyanilin), 3,3'-Dimethyl-4,4'-diaminobenzophenon, 3,3'-Dimethoxy-4,4'-diaminobenzophenon, 4,4'-Methylendianilin, 3,3'-Dimethylbenzidin, 3,3'-Dimethoxybenzidin, Diaminotoluol, 5-Amino-1-(4-aminophenyl)-1,3,3-trimethylindan.

$X^1$ als von X verschiedener Diaminrest kann z.B. unsubstituiertes oder durch Halogen oder $C_1$—$C_4$-Acyl substituiertes Phenylen oder Bisphenylen der Formel

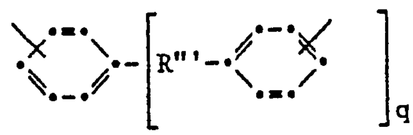

sein, worin q Null oder die Zahl 1 ist und R''' eine direkte Bindung, —O—, —S—, —SS—, —SO—, —$SO_2$—, —CO—,

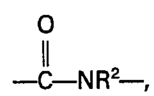

—COO—, —$NR^2$—, —NH—, —CONH—, —$SiR^3R^4$— oder —$OSi(R^3R^4)O$— bedeutet und $R^2$, $R^3$ und $R^4$ $C_1$—$C_6$-Alkyl, Phenyl oder Benzyl und $R^3$ und $R^4$ auch $C_1$—$C_6$-Alkoxy, Phenyloxy oder Benzyloxy sind. Die freien Bindungen befinden sich bevorzugt in Parastellung und besonders in Metastellung zur R'''-Gruppe. Beispiele für solche Diamine sind: 3,3'-Dichlorbenzidin, 3,3'-Sulfonyldianilin, 4,4'-oder 3,3'-Diaminobenzophenon, 1,5-Diaminonaphthalin, Bis-(4- oder 3-aminophenyl)-dimethylsilan, Bis-(4- oder 3-aminophenyloxy)dimethylsilan, N-Bis(4-aminophenyl)-N-methyl- oder —N-Phenylamin, 4,4'-Oxybis(2-chloranilin), 5,5'-Oxybis(2-aminophenol), 4,4'-Thiobis(anilin), 4,4'-Sulfonylbis(2-chloranilin), 5,5'-Sulfonylbis(2-aminophenol), 3,3'-Dichlor-4,4'-diaminobenzophenon, 4,4'- oder 3,3'-Diaminobisphenyl, m-Phenylendiamin, p-Phenylendiamin, 4,4'-Oxydianilin, 4,4'- oder 3,3'-Thiodianilin, 4,4'-Sulfonyldianilin, 3,3'-Dicarboxybenzidin.

Es ist bekannt, dass einige aliphatische und aromatische Diamine, z.B. Phenylendiamin oder Di(aminophenyl)methan, die Unlöslichkeit von Polyimiden fördern können. Solche Diamine werden daher bevorzugt in geringeren Mengen eingesetzt. Insbesondere sind für diesen Fall die Strukturelemente der Formel I zu mindestens 50 Mol-%, besonders 80 Mol-% und ganz besonders 90 Mol-% enthalten.

Die erfindungsgemäss zu verwendenden Polyimide weisen mittlere Molekulargewichte (Gewichtsmittel Mw) von bevorzugt mindestens 2000, besonders mindestens 5000 auf. Die obere Grenze richtet sich im wesentlichen nach Eigenschaften, die die Verarbeitbarkeit bestimmen, wie z.B. deren Löslichkeit. Sie kann bis zu 500 000, vorzugsweise bis zu 100 000 und besonders bis zu 60 000 betragen. Es kann sich ferner um statistische Polyimide oder um Blockpolyimide handeln. Sie werden nach üblichen Verfahren in hierfür vorgesehenen Einrichtungen hergestellt.

Die erfindungsgemäss zu verwendenden Polyimide können hergestellt werden, in dem man mindestens 0,1 Mol-% mindestens einer Tetracarbonsäure der Formel X

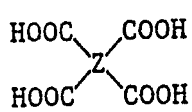 (X)

alleine oder zusammen mit höchstens 99,9 Mol-%, bezogen auf die Tetracarbonsäuren, mindestens einer Tetracarbonsäure der Formel Xa, und/oder Aminodicarbonsäure der Formel Xb oder deren polyimidbildenden Derivate,

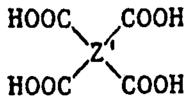 (Xa), 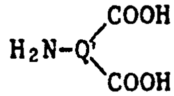 (Xb)

mit mindestens 0,1 Mol-%, bezogen auf die Diamine, mindestens eines Diamines der Formel XI

$$H_2N—X—NH_2 \quad (XI)$$

alleine oder zusammen mit höchstens 99,9 Mol-% mindestens eines Diamines der Formel XIa

$$HN_2—X'—NH_2 \quad (XIa)$$

in an sich bekannter Weise polykondensiert und anschliessend cyclisiert.

Die Diamine der Formeln XI und XIa sind bekannt oder nach bekannten Verfahren herstellbar. Sihaltige Diamine sind in der US—PS 3,435,002 und der EP—A—0 054 426 beschrieben. Diamine mit der

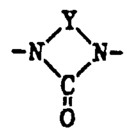

Gruppe können aus den in der DE—A—2 318 170 beschriebenen Diisocyanaten hergestellt werden. Alkyl- oder Cycloalkyl-substituierte, besonders Aethyl- oder Propyl-substituierte Diamine sind durch Alkylierungen von unsubstituierten oder teilsubstituierten aromatischen Diaminen mit Alkenen bzw. Cycloalkenen zugänglich (vgl. US—PS—3 275 690). Mehrkernige, besonders zweikernige aromatische Diamine sind über die Kondensation entsprechender Monoamine mit Aldehyden oder Ketonen erhältlich.

Die Tetracarbonsäuren der Formel Xa und Amindicarbonsäuren der Formel Xb sind ebenfalls bekannt. Tetracarbonsäuren mit einem Strukturelement der Formel VII sind in Macromol. Chem. 183, 1615—1622 (1982) beschrieben.

4,4'-Ketobis-(naphthalin-1,2-dicarbonsäure) ist durch die Umsetzung von 2 Mol 1,2-Dimethylnaphthalin mit $COCl_2$ in Gegenwart von $AlCl_3$ und die anschliessende Oxidation des gebildeten 4,4'-Ketobis(1,2-dimethylnaphthalins) mit $HNO_3$ zur Tetracarbonsäure erhältlich. Diese Tetracarbonbsäure kann partiell zum 4,4'-Keto-(tetrahydronaphthalin-1,2-dicarbonsäure) hydriert werden. Die Hydrierung kann auch von der Oxidation mit 4,4'-Ketobis(1,2-dimethylnaphthalin) vorgenommen werden.

Tetracarbonsäuren mit Strukturelementen der Formel IV sind teilweise bekannt.

Anthrachinontetracarbonsäuren sind z.B. im US-Patent 3 702 318 und CA 100,1006119a (1984) beschrieben.

Durch Hydrierung der Ketogruppen in Anthrachinontetracarbonsäuren mit z.B. $NaBH_4$ erhält man die entsprechende Dihydroxyverbindung, die durch Behandlung mit Salzsäure am Rückfluss in Anthrontetracarbonsäure überführt werden kann. Die $CH_2$-Gruppe der Anthrontetracarbonsäure kann in bekannter Weise alkyliert und so in die $CRR^1$-Gruppe in der Formel IV überführt werden.

Zur Herstellung von Fluorenontetracarbonsäure kann man z.B. 3,4-Dimethylmagnesiumbromid in Gegenwart von $CuCl_2$ dimerisieren und das gebildete 3,3',4,4'-Tetramethylbiphenyl mit $COCl_2$ in Gegenwart von $AlCl_3$ zum Tetramethylfluorenon umsetzen, das in bekannter Weise mi z.B. $HNO_3$ zu Tetracarbonsäure oxidiert werden kann.

Xanthontetracarbonsäure erhält man, indem man zunächst 3,3',4,4'-Tetramethylphenyläther mit $CCl_4$ in Gegenwart von $AlCl_3$ und anschliessender Hydrolyse mit verdünnter HCl zu 2,3,6,7-Tetramethylxanthon umsetzt, das in üblicher Weise zur Tetracarbonsäure oxidiert wird, z.B. mit $HNO_3$.

Tetracarbonsäuren mit Strukturelementen der Formel IV, worin Y S, SO, $SO_2$ oder NR bedeutet, sind auf folgendem Weg erhältlich: 1-Chlor-3,4-dimethylbenzol wird mit Oxalylchlorid in Gegenwart von $AlCl_3$ zum Bis-(5-chlor-2,3-dimethylphenyl)keton umgesetzt.

Die Oxidation mit 20%-iger $HNO_3$ führt zu

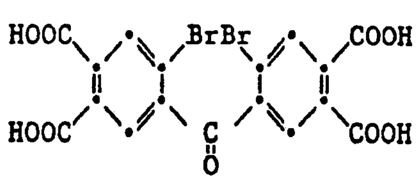 .

Die Umsetzung mit $Na_2S$ nach der Veresterung ergibt die Thioxanthontetracarbonsäure, die in bekannter Weise zum Sulfoxid bzw. Sulfon oxidiert werden kann. Die Umsetzung mit $RNH_2$ führt zu den Acridontetracarbonsäuren.

Ein weiterer Gegenstand vorliegender Erfindung sind Tetracarbonsären der Formel XII

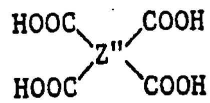

(XII),

worin Z'' ein Rest der Formeln XIIIa bis XIIIc

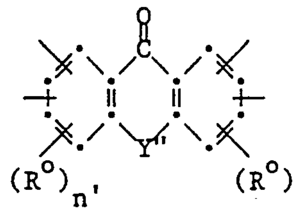

(XIIIa) ,

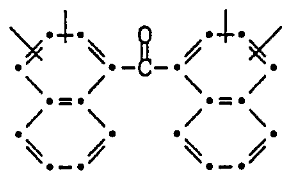

(XIIIb),

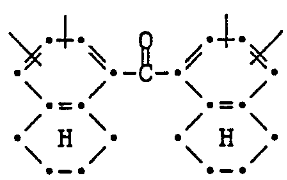

(XIIIc)

ist, in denen die freien Bindungen in Orthostellung zueinander gebunden sind, und Y'' eine direkte Bindung, $—CH_2—$, $—CH_2—CH_2—$, —O—, —S—, —SO—, $—SO_2—$, —NR— oder $—CRR^1—$ bedeutet, worin R ein Wasserstoffatom, $C_1—C_{10}$-Alkyl, Phenyl, Naphthyl oder Phenyl($C_aH_{2a}$)— mit a = 1—4 und $R^1$ die Bedeutung von R mit der Ausnahme der bedeutung eines Wasserstoffatomes hat und $R^o$ für $C_1—C_{10}$-Alkyl, Halogen, —CN, $—NO_2$, $C_1—C_{12}$-Alkoxy, Phenoxy, Naphthoxy oder Phenyl($C_aH_{2a}$)— mit a = 1—4 steht, und n' Null oder die Zahl 1 oder 2 ist, sowie die Säurederivate.

Säurederivate sind z.B. die Anhydride, Ester, Amide und Halogenide, besonders Chloride.

Bevorzugte Tetracarbonsäure der Formel XII sind solche, in denen in den Formeln XIIIa bis XIIIc die freien Bindungen in Meta- und Parastellung zur CO-Gruppe gebunden sind, n' Null ist und Y'' eine direkte Bindung, $—CH_2—$, —O—, —S—, —SO—, $—SO_2—$ ist.

Die Herstellung der Polyimide wird vorteilhaft in Lösung durchgeführt; geeignete inerte Lösungsmittel sind nachfolgend genannt. Die Reaktionstemperaturen können −20 bis 300°C betragen.

Im einzelnen geht man zweckmässig so vor, dass man Tetracarbonsäuredianhydrid und Diamin zunächst unter Bildung einer Polyamidsäurevorstufe umsetzt und diese Polyamidsäure anschliessend unter Wasserabspaltung cyclisiert. Die Cyclisierung kann thermisch erfolgen. Vorteilhaft wird die Cyclisierung unter Einwirkung von wasserentziehenden Mitteln, z.B. Carbonsäureanhydriden wie Acetanhyrid, vorgenommen. Die Polyimide können anschliessend nach üblichen Verfahren, z.B. durch Entfernen des Lösungsmittels oder Ausfällen durch Zugabe eines Nichtlösungsmittels, isoliert werden.

Eine weitere Herstellungsmethode besteht darin, dass man das Tetracarbonsäuredianhydrid mit einem Diisocyanat in einer Stufe zum Polyimid umsetzt.

Die erfindungsgemäss zu verwendenden Polyimide sind in unterschiedlichen Lösungsmitteln löslich, gegebenenfalls unter Erwärmen, und sie weisen hohe Glasumwandlungstemperaturen auf. Sie eignen sich hervorragend zur Herstellung von Filmen und Schutzüberzügen, wobei Beschichtungsmittel aus einer Lösung des Polyimides in einem Lösungsmittel Anwendung finden können.

Zur Herstellung des beschichteten Materials löst man das Polymer oder Gemische davon zweckmässig in einem geeigneten organischen Lösungsmittel, gegebenenfalls unter Erwärmung. Geeignete Lösungsmittel sind z.B. polare, aprotische Lösungsmittel, die alleine oder in Mischungen aus mindestens zwei Lösungsmitteln verwendet werden können. Beispiele sind: Aether wie Dibutyläther, Tetrahydrofuran, Dioxan, Methylenglykol, Dimethyläthylenglykol, Dimethyldiäthylenglykol, Diäthyldiäthylenglykol, Dimethyltriäthylenglykol, halogenierte Kohlenwasserstoffe wie Methylenchlorid, Chloroform, 1,2-Dichloräthan, 1,1,1-Trichloräthan, 1,1,2,2-Tetrachloräthan, Carbonsäureester und Lactose wie Essigsäureäthylester, Propionsäuremethylester, Benzoesäureäthylester, 2-Methoxyäthylacetat, γ-Butyrolacton, o-Valerolacten und Pivalolacton, Carbonsäureamide und Lactame wie Formamid, Acetamid, N-Methylformamid, N,N-Dimethylformamid, N,N-Diäthylformamid, N,N-Dimethylacetamid, N,N-Diäthylacetamid, γ-Butyrolactam, ε-Caprolactam, N-Methylpyrrolidon, N-Acetylpyrrolidon, N-Methylcaprolactam, tetramethylharnstoff, Hexamethylphosphortrisäureamid, Sulfoxide wie Dimethylsulfoxid, Sulfone wie Dimethylsulfon, Diäthylsulfon, Trimethylsulfon, Tetramethylensulfon, Amine wie Trimethylamin, Triäthylamin, N-Methylpiperidin, N-Methylmorpholin und substituierte Benzole wie Chlorbenzol, Nitrobenzol, Phenole oder Kresole.

Ungelöste Anteile können durch Filtration, bevorzugt einer Druckfiltration entfernt werden. Die Konzentration an Polymer im so erhaltenen Beschichtungsmittel beträgt vorzugsweise nicht mehr als 50 Gew.-%, besonders nicht mehr als 30 Gew.-% und insbesonders nicht mehr als 20 Gew.-%, bezogen auf die Lösung.

Bei der Herstellung der Lösung können weitere übliche Zusatzstoffe einverleibt werden, die die Lichtempfindlichkeit nicht negativ beeinflussen. Beispiele hierfür sind Mattierungsmittel, Verlaufmittel, feinteilige Füllstoffe, Flammschutzmittel, optische Aufheller, Antioxidantien, Lichtschutzmittel, Stabilisatoren, Farbstoffe, Pigmente, Haftvermittler und Antihalofarbstoffe, wie sie z.B. in der US—PS 4 349 619 beschrieben sind.

Das Beschichtungsmittel kann mittels üblichen Methoden wie Tauch-, Streich- und Sprühverfahren, Schleuder-, Kaskadenguss- und Vorhanggussbeschichtung, auf geeignete Substrate bzw. Trägermaterialien, aufgebracht werden, wobei Haftvermittler oder Klebschichten mit-verwendet werden können. Geeignete Substrate sind z.B. Kunststoffe, Metalle und Metallegierungen, Halbmettalle, Halbleiter, Glas, Keramik und andere anorganische Materialien wie z.B. $SiO_2$ und $Si_3N_4$. Danach wird das Lösungsmittel gegebenenfalls durch Erwärmen und gegebenenfalls im Vakuum entfernt. Man erhält klebfreie, trockene und gleichmässige Filme. Die aufgebrachten Filme können je nach Anwendung Schichtdicken bis zu ca.500 µm und mehr, bevorzugt von 0,5 bis 500 µm und besonders von 1 bis 50 µm aufweisen.

Es wurde gefunden, dass die erfindungsgemäss zu verwendenden Polyimide autophotovernetzbar sind und unter Strahlungseinwirkung vernetzt werden können.

Da die Lichtempfindlichkeit mit steigendem Gehalt an solchen Strukturelementen der Formel I steigt, ist ein Gehalt von mindestens 50 Mol-%, bevorzugt mindestens 80 Mol-% und besonders mindestens 90 Mol-% vorteilhaft.

Schutzfilme aus solchen Polyimiden können durch Strahlungseinwirkung weiter modifiziert werden, womit z.B. erhöhte Thermostabilitäten möglich sind. Ferner besteht die Möglichkeit, solche Polyimide als photographisches Aufzeichnungsmaterial für Reliefabbildungen einzusetzen. Durch die Direktvernetzung unter Strahlungseinwirkung können Zusätze wie Sensibilisatoren vermieden werden und die Schutzschichten und Abbildungen weisen ausgezeichnete elektrische Eigenschaften auf. Weiter zeichnen sich die Schutzschichten und Abbildungen durch ihre hohe Thermostabilität aus sowie durch keinen oder nur einen geringen Schwund bei thermischer Belastung, was in der Anwendung erhebliche Vorteile hat, weil praktisch keine Verzerrung abgebildeter Strukturen beobachtet wird.

Die Schichtdicke der Schutzschichten und photographischen Reliefabbildungen beträgt für diese Anwendung bevorzugt 0,5 bis 100 µm, besonders 1 bis 50 µm und insbesondere 1—10 µm.

Die Photostrucktturierung bzw. Photovernetzung kann durch energiereiche Strahlung hervorgerufen werden, z.B. durch Licht insbesondere im UV-Bereich, durch Röntgenstrahlen, Laerlicht, Elektronenstrahlen usw. Das erfindungsgemässe Material eignet sich hervorragend zur Herstellung von Schutzfilmen, Passivierlacken, und als photographisches Aufzeichnungsmaterial für thermostabile Reliefabbildungen.

Anwendungsgebiete sind z.B. Schutz-, Isolier- und Passivierlacke in der Elektrotechnik und Elektronik, Photomasken für die Elektronik, den Textildruck und das graphische Gewerbe, Aetzresist zur Herstellung gedruckter Schaltungen und Druckplatten und integrierter Schaltkreise, Realis für die Herstellung von Röntgenmasken, als Lötstopplack, als Dielektrikum für Mehrlagenschaltungen, als Strukturelement für Flüssigkristallanzeiger.

Die Herstellung von Schutzfilmen erfolgt durch direktes Belichten, wobei sich die Belichtungszeiten im wesentlichen nach den Schichtdicken und der Lichtempfindlichkeit richten.

Die photographische Erzeugung der Reliefstruktur erfolgt durch bildmässige Belichtung durch eine Photomaske, anschliessende Entwicklung unter Entefernung der unbelichteten Anteile mit einem Lösungsmittel oder einem Lösungsmittelgemisch, wonach gegebenefalls das erzeugte Bild durch eine thermische Nachbehandlung stabilisiert werden kann.

Als Entwickler sind z.B. die zuvor erwähnten Lösungsmittel geeignet.

Die polymerschicht des erfindungsgemäss zu verwendenden Materials weit eine für viele Anwendungszwecke ausreichende und teilweise hohe Lichtempfindlichkeit auf und sie kann direkt photovernetzt werden. Die Schutzfilme und Reliefabbildungen zeichnen sich durch hohe Haftfestigkeit und thermische, mechanische und chemische Widerstandsfähigkeit aus. Bei thermischen Nachbehandlungen wird nur ein geringer Schwund beobachtet. Ferner können Zusätze zur Erzeugung bzw. Steigerung der Lichtempfindlichkeit vermieden werden. Das Material ist lagerstabil, aber vorteilhaft vor Lichteinwirkung zu schützen.

Die nachfolgenden Beispiele erläutern die Erfindung näher.

Beispiel a

Herstellung von Xanthontetracarbonsäuredianhydrid

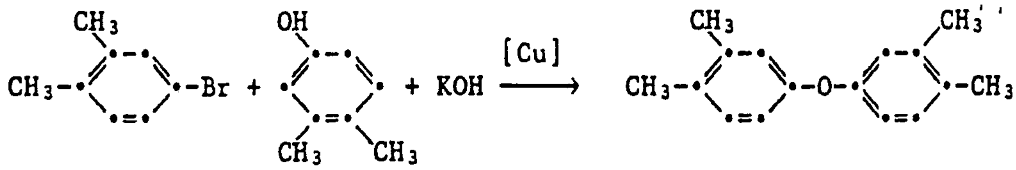

(A)

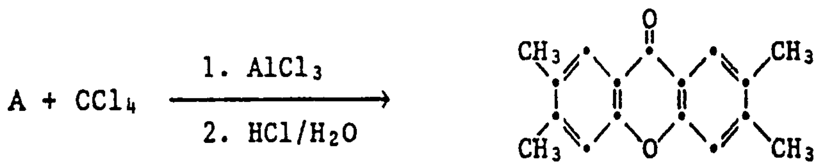

(B)

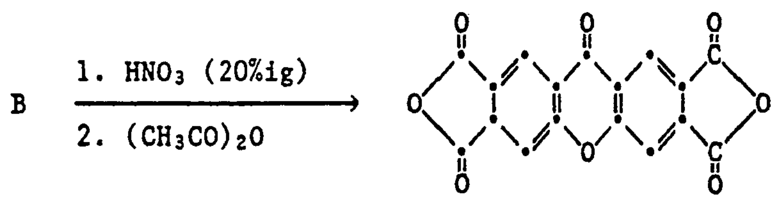

(C)

Herstellung von A

Eine Mischung von 165 g 4-Bromo-o-xylol, 122 g 3,4-Dimethylphenol, 66 g KOH und 10 g Cu-Pulver wird in einem Kolben auf 170°C erhitzt und über Nacht bei dieser Temperatur gehalten. Am nächsten Morgen werden weitere 10 g Cu-Pulver zugegeben und die Mischung auf 200°C gehalten, bis etwa 10 ml $H_2O$ abdestilliert sind und die Mischung erstarrt. Nach weiteren 2 Stunden bei 240°C wird abgekühlt, mit Wasser versetzt und mit Chloroform extrahiert. Nach Abdestillileren des Lösungsmittels wird der Rückstand destilliert. Dabei werden 127 g A erhalten mit einem Siedepunkt von 175—178°C bei 18,2 mbar.

Elementaranalyse:

| | berechnet | gefunden |
|---|---|---|
| C | 84,92 | 85,04 |
| H | 8,02 | 7,97 |
| O | 7,07 | 7,25 |

Herstellung von B

22,6 g A, in $CCl_4$ gelöst, werden zu einer Mischung von 26,6 g $AlCl_3$ und 50 ml $CCl_4$ bei Raumtemperatur langsam zugetropft. Während die HCl-Entwicklung einsetzt, steigt die Temperatur auf 40°C. Nach 3 Stunden wird das Reaktionsgemisch auf Eiswasser gegossen, mit $CHCl_3$ verdünnt und die organische Phase abgetrennt. Nach Abdestillieren des Lösungsmittels wird der Rückstand mit 250 ml 15%-iger Salzsäure versetzt und 24 Stunden unter Rückfluss gekocht.

Das feste Produkt wird abfiltriert, mit Wasser gewaschen und anschliessend mit Aether verrührt. nach erneuter Filtration wird der Rückstand getrocknet und ohne weitere Reinigung für die Herstellung von C eingesetzt.

Herstellung von C

15 g B werden in einem Rührautokalven zusammen mit 700 ml 20-%-iger Salpetersäure 10 Stunden bei 180°C gehalten. Die abgekühlte Lösung wird zur Trockene eingedampft und der Rückstand (18,2 g) mit

400 ml Acetanhydrid 24 Stunden am Rückfluss erhitzt. Die gebildete Essigsäure und das überschüssige Acetanhydrid wird abdestilliert und der Rückstand bei 300°C im Hockvakuum (<1 mbar) sublimiert.

Elementaranalyse:

| | berechnet | gefunden |
|---|---|---|
| C | 60;73 | 60,10 |
| H | 1,20 | 1,16 |
| O | 38,07 | 37,93 |

Beispiel b

Herstellung von Thioxanthontetracarbonsäuredianhydrid

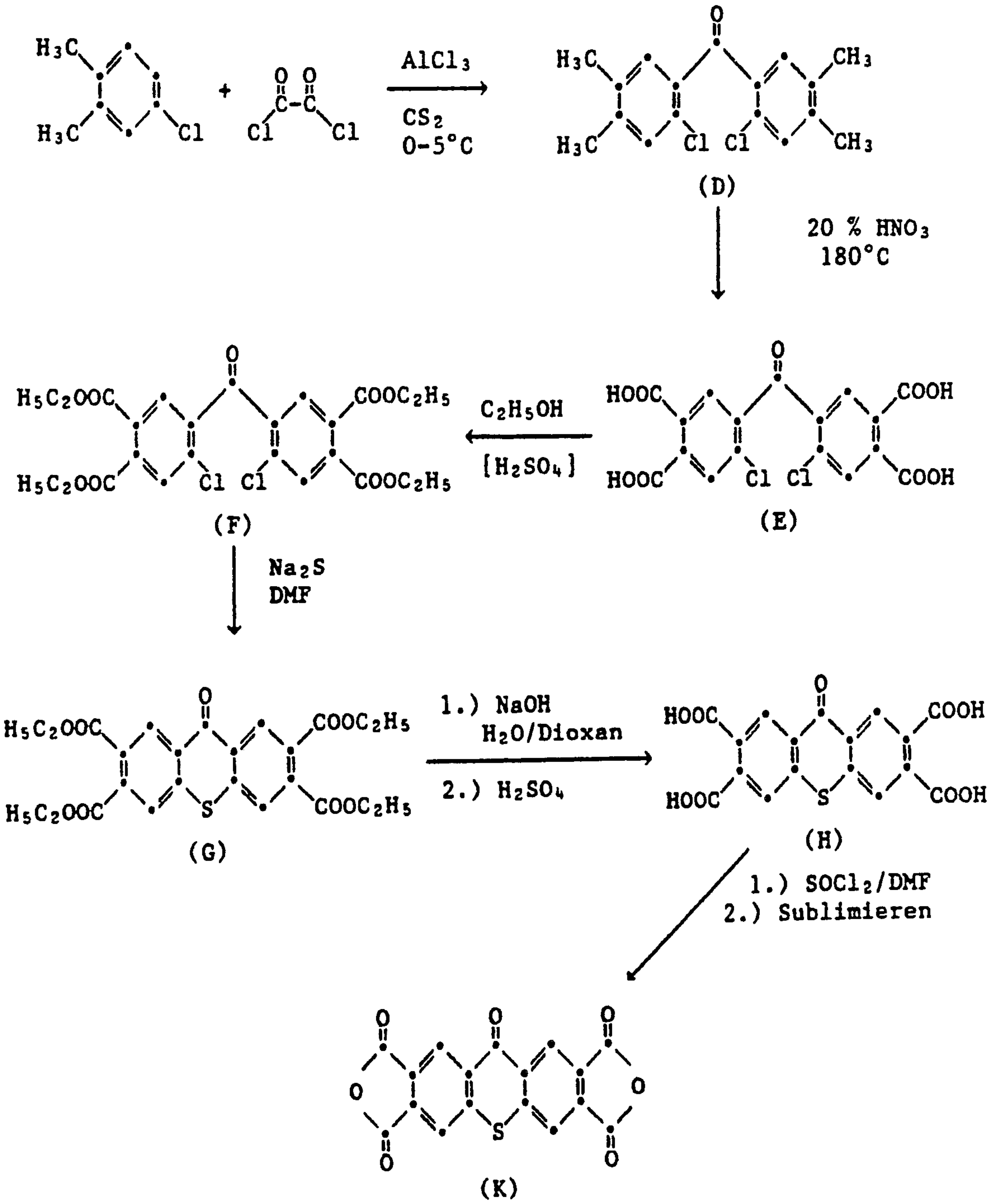

Herstellung von D

Zu einer Lösung von 140 g 4-Chlor-o-xylol und 68 g Oxalychlorid in 500 ml $CS_2$ werden bei 0—5° während 2 Stunden unter Rühren 146 g $AlCl_3$ zugegeben. Nach 20 Stunden wird das Gemisch auf Eis gegossen un mit 600 ml $CHCl_3$ extrahiert. Der Extrakt wird mit Wasser gewaschen, 'getrocknet und vollständig eingedampft. Die Umkristallisation des Rückstandes aus Diisopropyläther gibt 103,8 g 2,2'-Dichlor-4,4',5,5'-tetramethylbenzophenon (D).

Schmelzpunkt: 148—149°C

Elementaranalyse:

| berechnet | gefunden |
|---|---|
| C 66,46% | C 66,8% |
| H 5,25% | H 5,5% |
| O 5,21% | O 5,2% |
| Cl 23,08% | Cl 33,1% |

Herstellung von E

76 g D in 1,5 1—20%iger Salpetersäure suspendiert, werden im Tantalautoklaven 24 Stunden lang auf 180°C erwärmt. Nach dem Abkühlen wird filtriert, der feste Rückstand mit Eiswasser gewaschen und getrocknet. Es werden 68 g Tetracarbonsäure (E) erhalten, die ohne weitere Reinigung weiterverarbeitet wird.

Herstellung von F

68 g E werden verestert, indem E 25 Stunden in 600 ml Aethanol und 15 ml $H_2SO_4$ konzentriert unter Rückfluss gekocht wird. Nach der üblichen Aufarbeitung wird der Teraäthylester (F) durch Säulenchromatographie an Kieselgel (Elution mit Toluol/$CH_2Cl_2$ 1:1 + 2% Diäthyläther) gereinigt und anschliessend zweimal aus Diisopropyläther umkristallisiert. Dabei werden 41,8 g F erhalten.

Schmelzpunkt: 95—96°C

Elementaranalyse:

| berechnet | gefunden |
|---|---|
| C 55,57% | C 55,6% |
| H 4,49% | H 4,4% |
| O 26,70% | O 26,6% |
| Cl 13,15% | Cl 13,1% |

Herstellung von G

Eine Mischung von 26,9 g F, 7,8 g wasserfreiem Natriumsulfid und 100 ml Dimethylformamid wird unter Inertgas 1 Stunde lang auf 120°C erwärmt und anschliessend auf 800 ml Eiswasser gegossen. Das ausgefällte Produkt wird filtriert und aus Diisopropyläther umkristallisiert. Dabei werden 31,7 g G erhalten.

Schemelzpunkt: 108—109°C

| berechnet | gefunden |
|---|---|
| C 59,99 | C 59,9 |
| H 4,83 | H 4,9 |
| O 28,77 | O 28,7 |
| S 6,41 | S 6,4 |

Herstellung von H

20 g Tetraäthylester (G) werden zur Tetracarbonsäure verseift indem die Substanz zuerst 5 Stunden in einem Gemisch von 200 ml 2 N NaOH und 50 ml Dioxan erwärmt wird und anschliessend mit verdünnter

$H_2SO_4$ angesäuert wird. Die ausgefallene Tetracarbonsäure wird abfiltiert (H) und getrocknet. Es werden 16,5 g H erhalten, die ohne weitere Reinigung weiterverarbeitet werden.

Herstellung von K

10,9, g Tetracarbonsäure (H) werden in das Dianhydrid (K) übergeführt durch 20-stündige Behandlung mit einer Mischung von 60 ml DMF und 13,32 g Thionylchlorid bei Raumtemperatur. Duch Zugabe von 100 ml Toluol wird das Dianhydrid ausgefällt und abfiltriert. Die getrocknete Substanz wird durch Sublimation im Hochvakuum bei 350°C gereinigt. Es werden 7,4 g Dianhydrid (K) erhalten.

Elementaranalyse:

| berechnet | gefunden |
|---|---|
| C 57,96 | C 57,7 |
| H 1,15 | H 1,3 |
| O 31,79 | O 32,1 |
| S 9,1 | S 8,9 |

Beispiel c

a) Thioxanthon-10-oxid-2,3,6,7-tetracarbonsäure-tetraäthylester

Zu einer gerührten Lösung von Thioxanthon-2,3,6,7-tetracarbonsäuretetraäthylester (20 g, gemäss Beispiel b) in 200 ml Dichloromethan werden bei −30°C bis −40°C innert einer Stunde 100 ml einer Lösung von Peroxytrifluoressigsäure in Dichlormethan (gemäss *A. S. Pagano und W. G. Emmons,* Organic Syntheses Coll. Vol. *V,* 367 (1973)) getropft. Nach 30 min Rühren bei −20°C wird auf Eiswasser gegossen, die organische Phase abegtrennt, mit 5-%ige Natriumkarbonat-Lösung und Wasser gewaschen, mit Magnesiumsulfat getrocknet und eingedampft. Der Rückstand (20 g) wird durch Säulenchromatographie an Silikagel aufgetrennt. Mit Dichloromethan/Acethylacetat = 20:1 weden zunächst 15,2 g 10,10-Dioxid, $R_f$-Wert 0,58, dann 4,8 g 10-Oxid, $R_f$-Wert 0,3, eluiert.

Schemlzpunkt: 154—155°C

Elementaranalyse:

| berechnet | gefunden |
|---|---|
| C 58,13% | C 58,0% |
| H 4,68% | H 4,9% |
| O 30,97% | O 31,2% |
| S 6,21% | S 6,1% |

b) Thioxanthan-10-oxid-2,3,6,7-tetracarbonsäure

Zu einer Lösung von 4 g Tretraäthylester gemäss a) in 20 ml Dioxan werden 80 ml 2 N-Natronlauge gegeben. Nach 4,5 Stunden Rühren bei 60°C wird auf Raumtemperatur abgekühlt und mit Aethylacetat extrahiert. Trocknen (Magnesiumsulfat) und Eindampfen der organischen Phase gibt 3,3 g tetracarbonsäure, welche ohne Reinigung weiter umgesetzt wird.

c)Thioxanthon-10-oxid-2,3,6,7-tetracarbonsäure-bis-anhydrid

Methode A

Zu einer Lösung von 400 mg Tetracarbonsäure gemäss b) in 3 ml Dimethylformamid werden 0,15 ml Thionylchlorid gegeben. Nach 20 Stunden Rühren bei Raumtemperatur werden 5 ml Toluol zugegeben und das Lösungsmittel wird im Vakuum abgedampft. Der Rückstand wird bei 509°C/0,026 mbar getrocknet, mit 1 ml Dioxan verrieben und filtriert. Trocknen des Filtergutes (50°C/0,026 mbar) gibt 130 mg bis-Anydrid.

Ifrarot-Spektrum (Suspension in Nujol): Banden mit Maxima bei 1885 $cm^{-1}$ und 1173 $cm^{-1}$.

Methode B

50 mg Tetracarbonsäure gemäss b) werden in 20 ml Xylol suspendiert. Während 2 Sunden wird unter Rühren in einem Heizbad von 140°C erwärmt und langsam destilliert. Es wird von ungelöstem Material abfiltriert, gekühlt und im Vakuum eingedampft.

Ausbeute 32 g bis-Anhydrid.

d) Lösung von Thioxanthon-10-oxid-2,3,6,7-tetracarbonsäure-tetra(trimethylsilyl)ester in Toluol

Zu einer Suspension von 100 mg Tetracarbonsäure gemäss b) in 25 ml Toluol werden 100 mg N,O-Bis-(trimethylsilyl)-acetamid gegeben. Nach kurzuem Rühren (ca. 5 min) entsteht eine klare Lösung von Tetra-(trimethylsilyl)ester, welche direkt für Polykondensationen verwendet werden kann.

Beispiel d

Thioxanthon-10,10-dioxid-2,3,6,7-tetracarbonsäure

Methode A

a) Thioxanthon-10,10-dioxid-tetracarbonsäure-tetraäthylester

Zu einer gerührten Lösung von 3 g Thioxanthon-2,3,6,7-tetracarbonsäure-tetraäthylester gemäss Beispiel b in 60 ml Dichloromethan werden bei 0—5°C 19,5 ml einer Lösung von Peroxytrifluoressigsäure in Dichloromethan (gemäss *A. S. Pagano und W. G. Emmons,* Organic Syntheses Coll. Vol. *V,* 367 (1973)) getropft. Die Mischung wird 3 Stunden unter Rückfluss zum Sieden erhitzt, gekühlt und mit 5-%iger Natriumkarbonat-Lösung sowie mit Wasser gewaschen. Eindampfen der getrockneten (Magnesiumsulfat) organischen Phase und Verreiben des Rückstandes (4,5 g) mit 10 ml Diisopropyläther gibt 3,1 g 10,10-Dioxid.

Schmelzpunkt: 136—137°C.

Elementaranalyse:

| berechnet | gefunden |
|---|---|
| C 56,38% | C 56,4% |
| H 4,54% | H 4,7% |
| O 33,05% | O 33,1% |
| S 6,02% | S 6,0% |

b) Thioxanthon-10,10-dioxid-2,3,6,7-tetracarbonsäure

Zu einer Lösung von 16 g Tetraäthylester gemäss a) in 50 ml Dioxan werden 200 ml 2 N-Natronlauge gegeben. Nach 7 Stunden Rühren bei 60°C wird abgekühlt, mit 10-%iger Schwefelsäure sauer gestellt und fünfmal mit je 150 ml Aethylacetat ausgeschüttelt. Trocknen der organischen Phase mit Mangesiumsulfat und Eindampfen gibt 10,9 g Tetracarbonsäure.

c) Methode B

Eine Suspension von 1 g Thioxanthon-2,3,6,7-tetracarbonsäure gemäss Beispiel b in 50 ml 20-%iger Salpetersäure wird 24 Stunden im verschlossenen Glasrohr unter Zugabe eines Tantalkörpers auf 180°C erwärmt. Eindampfen der Lösung, Lösen in 50 ml Wasser, Zugabe von 100 mg Aktivkohle, Filtration und Eindampfen des Filtrates gibt 1,1 g rohes 10,10-Dioxid.

Beispiel e

2,3,6,7-Tetracarboethoxy- und 1,2,6,7-Tetracarboethoxy-fluorenon

Methode A

a) 3,3',4,4'-Tetramethyl-biphenyl

Zu einer Suspension von Magnesium-Spänen (1,95 g) in 50 ml trockenem Tetrahydrofuran wird während 1 Stunde eine Lösung von 13,7 g 4-Bromn-o-Xylol in 50 ml trockenem Tetrahydrofuran getgropft. Nach 2 Stunden Kochen unter Rückfluss in einer Argon-Atosphäre wird auf Raumtemperatur gekühlt und portionenweise werden 9,4 g wasserfreies Kupfer(II)chlorid zugegeben. Man giesst auf Eiswasser und extrahiert zweimal mit je 100 ml Diäthyläther. Die organische Phase wird mit 2 N-Salzsäure und gesättigter Kochsalzlösung gewaschen. Trocknen mit Magnesiumsulfat, Eindampfen und Kristallisation des Rückstandes aus Aethanol gibt 2,9 g Biphenyl.

Schmelzpunkt: 74—76°C

Massenspektrum: m/e = 210 (100%, $M^+$).

b) 2,3,6,7-Tetramethyl- und 1,2,6,7-Tetramethyl-fluorenon

Zu einer Lösung von 20 g Biphenyl gemäss a) und 14 g Phosgen in 130 ml Schwefelkohlenstoff werden bei −10°C bis 0°C innert 2 Stunden 35,6 g wasserfreies Aluminiumchlorid portionenweise zugegeben. Nach 15 Stunden Rühren bei Raumtemperatur wird 2 Stunden unter Rückfluss zum Sieden erhitzt, auf Eiswasser gegossen und mit 1,5 l Chloroform extrahiert. Der Rückstand der organischen Phase (22,9 g) wird durch

Säulenchromatographie an Kieselgel aufgetrennt. Elution mit Dichloromethan/Toluol = 13:7 gibt 9,8 g 1,2,6,7-Tetramethylfluorenon ($R_f$-Wert 0,62) und 10,04 g 2,3,6,7-Tetramethyl-flourenon ($R_f$-Wert 0,41).

2,3,6,7-Tetramethyl-fluorenon:

Schmelzpunkt (Toluol/Ligroin): 254—256°C.

Elementaranalyse:

| berechnet | gefunden |
|---|---|
| C 86,41% | C 86,3% |
| H 6,83% | H 6,7% |
| O 6,77% | O 6,8% |

1,2,6,7-Tetramethyl-fluorenon:

Schmelzpunkt (Toluol/Ligroin): 263—264°C

Elementaranalyse:

| berechnet | gefunden |
|---|---|
| C 86,41% | C 86,5% |
| H 6,83% | H 7,1% |
| O 6,77% | O 6,9% |

Methode B

c) 3,4-Dimethyl-benzoesäure-anhydrid

Zu einer Mischung von 96.1 g 3,4-Dimethyl-benzoesäure und 108,1 g Dimethyl-benzoylchlorid in 600 ml Dichloromethan werden unter Eiskühlung 88,6 ml Triäthylamin getropft. Nach 3,5-Stunden Rühren bei Raumptemperatur wird filtriert. Eindampfen des Filtrates und Kristallisation des Rückstandes aus Toluol gibt 139 g Anhydrid.

Schmelzpunkt: 114—115°C.

Infrarot-Spektrum (3% in Chloroform): Banden mit Maxima bei 1780 $cm^{-1}$ und 1715 $cm^{-1}$.

d) 2,3,6,-Tetramethyl- und 1,2,6,7-Tetramethyl-fluorenon Eine Mischung von 6,96 g Anhydrid gemäss c) und 300 mg tris-Triphenylphosphin-Rhodium(I)chlorid wird in einer Argon-Atmosphäre während 6 Stunden in einem Heizbad von 240°C erwärmt. Nach dem Kühlen wird in Chloroform aufgenommen, mit 10-%iger Natronlauge gewaschen, getrocknet (Magnesiumsulfat) und eingedampft. Säulenchromatographie des Rückstandes gemäss b) gibt 0,3 g 1,2,6,7-Tetramethyl-fluorenon und 0,94 g 2,3,6,7-Tetramethyl-fluorenon.

e) Fluorenon-2,3,6,7-tetracarbonsäure-tetraäthylester Eine Suspension von 0.7 g Tetramethyl-fluorenon in 60 ml 20-%iger Salpetersäure wird während 24 Stunden in geschlossenen Glasrohr unter Zagabe eines Tantalkörpers auf 180°C erwärmt. Die Lösung wird eingedampft, der Rückstand in 25 ml Wasser aufgenommen und filtriert. Das Filtrat wird eingedampft, der Rückstand (0,49 g) in 40 ml Aethanol gelöst und nach Zugabe von 0,1 ml konzentrierter Schwefelsäure wird 24 Stunden unter Rückfluss gekocht. Nach Zugabe von Wasser wird mit Chloroform extrahiert. Säulenchromatographie des Rückstandes der organischen Phase an Kieselgel mit Toluol/Diäthyläther = 20:1 als Elutonsmittel gibt 143 mg Tetraester ($R_f$-Wert: 0,068).

Schmelzpunkt (Dichloromethan/Hexan): 167—168°C.

Elementaranaylse:

| berechnet | gefunden |
|---|---|
| C 64,10% | C 64,0% |
| H 5,16% | H 5,3% |
| O 30,74% | O 30,6% |

Beispiel 1:

In einem zylindrischen Gefäss mit Rührer, Tropftrichter, Innenthermometer, Gaseinleitungsrohr und Gasableitungsrohr werden 0,847 g (0,003 Mol) 4,4'-Diamino-3,3'-dimethyl-5,5'-diäthyldiphenylmethan in

10 ml N-Methylpyrrolidon (NMP) gelöst und auf 0°C abgekühlt unter gleichzeitigem Einleiten von Stickstoff. Zu dieser Lösung werden insgesamt 1,018 g 0.00303 Mol) Xanthontetracarbonsäuredianhydrid in 4 Portionen (0,958 g, 0,03 g, 0,02 g, 0,01 g) im Verlaufe von 18 Stunden zugegeben. Zwei Stunden nach der letzten Zugabe werden bei Rautemperatur 0,9 ml Triäthylamin und 2,55 ml Essigsäureanhydrid zugegeben und 24 Stunden lang gerührt.

Dann wird die Polymerlösung auf 1 Liter Wasser gegossen und das Polyimid ausgefällt, filtriert und mit Wasser gewaschen. Nach Trocknung im Vakuum bei 80°C erhält man 1,7 g Polyimid. Die inhärente Viskosität, gemessen als 0,5-prozentige Lösung in NMP beträgt 0,186 dl/g.

Beispiel 2

Beispiel 1 wird wiederholt mit dem Unterschied, dass 33,3 Mol-% des dort verwendeten Diamins durch 3,6-Diaminodurol ersetzt werden.

Beispiel 3

Beispiel 2 wird wiederholt mit dem Unterschied, dass anstelle des dort verwendeten Diamins ein Diamin der Formel

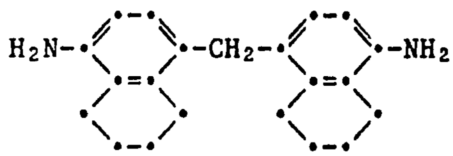

eingesetzt wird. Die inhärente Viskosität beträgt 0,114 dl/g.

Applikationsbeispiel

Auf einer einseitig kupferkaschierten Kunststoffplatte wird ein dünner Polymerfilm erzeugt, indem eine 10 prozentige Polymerlösung in NMP aufgeschleudert und dans Lösungmittel anschliesssend im Umluftofen entfernt wird.

Die so beschichtete Platte wird durch eine Photomaske (Stouffer-Keil) bei Raumtemperatur mit einer 1000-Watt-UV-Lampe aus 18 cm Entfernung belichtet. Die belichtete Platte wird anschliessend mit NMP entwickelt, wobei die unbelichteten Stellen des Polymerfilms weggelöst werden. Die Sichtbarmachung des Reliefbildes geschieht anschliessend durch Wegätzen der freigelegten Kupferschicht mit $FeCl_3$-Lösung.

Die Photoempfindlichkeit wird nach dem "21-step Stouffer sensitivity guide" ermittelt und beträgt für die Polymeren aus den Beispielen 1, 2 und 3:

| Beispiel | Belichtungszeit | letzte abgebildete Stufe |
|---|---|---|
| 1 | 120 sec | 6 |
| 2 | 240 sec | 8 |
| 3 | 420 sec | 4 |

Beispiel 4

Analog Beispiel 1 wird ein Mischung von 2,4-Diamino-3,5-diäthyltoluol und 4,4'-Diamino-3,3',5,5'-tetramethyldiphenylmethan (1:1) mit einer äquivalenten Menge Anthrachinon-2,3,6,7-tetracarbonsäuredianhydrid umgesetz und mit Triäthylamin und Essigsäureanhydrid zum Polyimid cyclisiert.

Die inh. Lösungsviskosität beträgt 0,89 dl/g. Die Glasumwandlungstemperatur, gemessen im Differentialscanningcalometric (DSC), beträgt 427°C. Die Photoempfindlichkeit gemäss Verfahrem im Applikationsbeispiel beträgt 5-6 Stufen bei einer Belichtungszeit von 30 sec.

Beispiel 5

Eine Diaminmischung, wie in Beipiel 2 beschrieben, wird analog Beispiel 1 mit der äquivalenten Menge 4,4'-Ketodinaphthalsäuredianhydrid umgesetzt und zum Polyimid cyclisiert.

Das Polyimid eignet ebenfalls zur photographischen Reliefbilderzeugung bei Bestrahlung mit UV-Licht.

Beispiel 6

In einem Kolben mit Rührer und Destillationsaufsatz werden 1,64 g 3,6-Diaminodurol und 0,285g 3,4-Dicarboxy-3'-aminobenzophenon in 30 ml N-Methylpyrrolidon (NMP) gelöst und auf Siedetemperatur erhitzt. Im Verlaufe von etwa 30 min wird das Reaktionswasser zusammen mit ca. 10 ml NMP abdestilliert. Zur erkalteten Lösung werden 3,22 g Benzophenontetracarbonsäuredianhydrid (BTDA) gegeben und die Lösung 5 Stunden gerührt. Nach Zugabe von weiteren 32,2 mg BTDA wird noch zwei Stunden gerührt. Die

Lösung wird dann mit einer Mischung von 3 ml Triäthylamin und 8,5 ml Acetanhydrid versetzt. Nach 16 Stunden ständigem Rühren wird das Polyimid durch Eingiessen der Lösung in Wasser ausgefällt und filtriert, mit Wasser gewaschen und getrocknet.

Inhärente Viskosität: 0,52 dl/g.

Glasumwandlungstemperature (DSC): 415°C.

Die Photoempfindlichkeit gemäss Applikationsbeispiel beträgt 7.

Stufen bei einer Belichtungszeit von 60 sec.

Beispiel 7

Gemäss Beispiel 1 wird eine Mischung von 0,651 g 2,4-Diamino-3,5-diäthyltoluoldiamin (0,0036 Mol) und 2,400 g 4,4'-Diamino-3,3-dimethyl-5,5'-diäthyldiphenylmethan (0,0085 Mol) in 29,5 ml NMP mit 4,278 g Thioxanthontetracarbonsäuredianhydrid (0,0124 Mol) zur Polyamidsäure umgesetzt und anschliessend nach Zugabe von weiteren 20 ml NMP mit Acetanhydrid und Triäthylamin zum Polyimid cyclisiert.

Inhärente Lösungsviskosität: 0,79 dl/g.

Glasumwandlungstemperatur: 397°C.

Die Photoempfindlichkeit (gemäss Applikationsbeispiel) beträgt.

Stufe 2 bei einer Belichtungszeit von 10 sec.

## Patentansprüche

1. Verfahren zur Herstellung von Schutzfilmen oder photographischen Reliefabbildungen aus Polyimiden, dadurch gekennzeichnet, dass man ein beschichtetes Material aus einem Trägermaterial, auf dem auf mindestens einer Oberfläche eine Schicht eines photovernetzbaren Homo- oder Copolyimids aus mindestens einer aromatischen Tetracarbonsäure und mindestens einem Diamin aufgebracht ist, wobei die Polyimide 0,1 bis 100 Mol-% mindestens eines Strukturelementes der Formel I

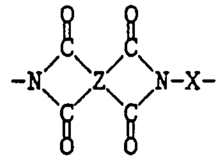

(I)

und 99,9 bis 0 Mol-% mindestens eines Struckurelementes der Formeln II und/oder III

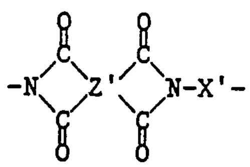

(II),

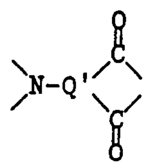

(III)

enthalten, worin

Z für mindestens einen vierwertigen Reste der Formeln IV, V, VI oder VII

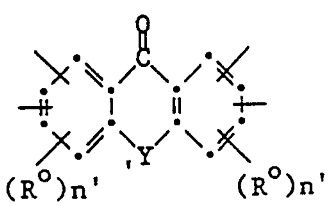

(IV),

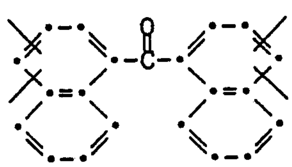

(V)

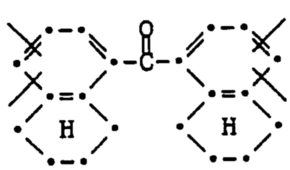

(VI)

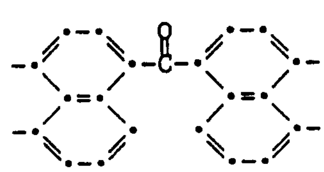

(VII)

steht, in denen die freien Bindungen in Orthostellung zueinander gebunden sind und Y eine direkte Bindung, —$CH_2$—, —$(CH_2)_2$—, —O—, —S—, —SO—, —$SO_2$—, —CO—, —NR— oder —$CRR^1$— bedeutet, wobei R ein Wasserstoffatom, $C_1$—$C_{10}$— Alkyl, Phenyl, Napthyl oder Phenyl($C_aH_{2a}$)— mit a = 1 bis 4 ist und $R^1$ die Bedeutung von R mit Ausnahme der Bedeutung eines Wasserstoffatomes hat, $R^0$ für $C_1$—$C_{10}$-Alkyl, Halogen, —CN, —$NO_2$, $C_1$—$Cl_2$-Alkoxy, Phenoxy, Naphthoxy oder Phenyl-($C_aH_{2a}$)- mit a = 1—4 steht, n' Null oder die Zahl 1 oder 2 ist,

X ein unsubstituierter oder substituierter zweiwertiger aliphatischer Rest, der durch Heteroatome, aromatische, heterocyclische oder cycloaliphatische Gruppen unterbrochen sein kann, ein unsubstituierter oder substituierter heterocyclischer, cycloaliphatischer oder araliphatischer Rest, ein aromatischer Ret, bei dem zwei Arylkerne über eine aliphatische Gruppe verknüpft sind, oder ein durch mindestens eine Alkylgruppe, Cycloalkylgruppe, Alkoxygruppe, Alkoxyalkylgruppe, Alkylthiogruppe, Alkylthioalkylgruppe, Hydroxyalkylgruppe, Hydroxyalkoxygruppe, Hydroxyalkylthiogruppe, Aralkylgruppe oder zwei benachbarte C-Atome des aromatischen Restes durch eine Alkylengruppe substituierter aromatischer Rest ist,

Q' ein dreiwertiger aromatischer Rest ist,

Z' die gleiche Bedeutung wie Z hat, oder ein von Z verschiedener vierwertiger organischer Rest ist, und

X' der zweiwertige, von X verschiedene Reste eines organischen Diamines ist,

wobei Z in Formel I auch vierwertige Benzophenonreste darstellen kann, wenn Strukturelemente der Formel III enthalten sind, flächenmässig oder unter einer Photomaske bestrahlt und die unbelichteten Anteile anschliessend mit einem Lösungsmittel entfernt.

2. Verfahren gemäss Anspruch 1, dadurch gekenzeichnet, dass man Polyimide verwendet, worin Y in Formel IV für eine direkte Bindung, —$CH_2$—, —O—, —S— oder —CO— steht.

3. Verahren gemäss Anspruch 1, dadurch gekennzeichnet, dass man Polyimide verwendet, worin in den Formeln IV bis VII die freien Bindungen in Meta- und Parastellung zur CO-Gruppe stehen.

4. Verfahren gemäss Anspruch 1, dadurch gekennzeichnet, dass man Polyimide verwendet, worin der Substituent des aromatischen Restes als Alkyl oder Alkoxy 1 bis 20 C-Atome, als Alkoxyalkyl 2 bis 12 C-Atome, als Cycloalkyl 5 oder 6 Ringkohlenstoffatome und als Alkylen 3 oder 4 C-Atome enthält und als Aralkyl Benzyl ist.

5. Verfahren gemäss Anspruch 4, dadurch gekennzeichnet, dass man Polyimide verwendet, worin der Substituent Alkyl mit 1 bis 4 C-Atomen, bevorzugt Isopropyl, Ethyl und besonders Methyl ist.

6. Verfahren gemäss Anspruch 1, dadurch gekennzeichnet, dass man Polyimide verwendet, worin Z' in Formel II als von Z verschiedener aromatischer Rest 6 bis 30 C-Atome enthält.

7. Verfahren gemäss Anspruch 6, dadurch gekennzeichnet, dass man Polyimide verwendet, worin Z' in Formel II ein Rest der Formeln

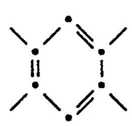

und besonders

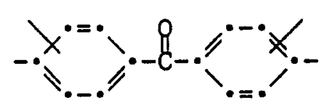

oder Mischungen davon ist.

8. Verfahren gemäss Anspruch 1, dadurch gekennzeichnet, dass man Polyimide verwendet, worin ein oder besonders zwei Substituenten im aromatischen Rest X in Formel I in Orthostellung zum N-Atom gebunden sind.

9. Verfahren gemäss Anspruch 1, dadurch gekennzeichnet, dass man Polyimide verwendet, worin X in Formel I als aliphatischer Rest 2 bis 30 C-Atome, als cycloaliphatischer Rest 5 bis 8 Ring-C-Atome, als araliphatischer Rest 7 bis 30 C-Atome und als substituierter aromatischer Rest 7 bis 30 C-Atome enthält.

10. Verfahren gemäss Anspruch 1, dadurch gekennzeichnet, dass man Polyimide verwendet, worin X in Formel I als substituierter aromatischer Rest den Formeln VIII, VIIIa und/oder VIIIb entspricht,

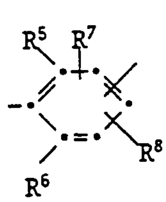

(VIII)

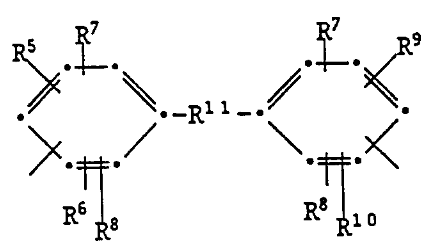

(VIIIa)

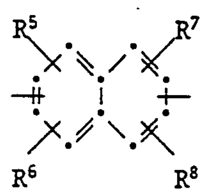

(VIIIb),

worin in Formel VIII die freien Bindungen in Meta- oder Parastellung zueinander stehen, in Formel VIIIa die freien Bindungen bevorzugt in Meta- oder Parastellung zur $R^{11}$-Gruppe stehen und $R^5$ und $R^6$ in den beiden Orthostellungen der freien Bindungen gebunden sind, und in Formel VIIIb die freien Bindungen in 2-, 3-, 6- und 7-Stellung gebunden sind und $R^5$ und $R^6$ sich in den beiden Orthostellungen der freien Bindungen befinden, $R^{11}$ für eine direkte Bindung, —O—, —S—, —SS—, —SO—, —$SO_2$—, —CO—, —COO—, —NH—,

|
—N-Alkyl

mit 1 bis 6 C-Atomen in Alkyl,

|
—N-Phenyl,

|
—N-Benzyl,

—CONH—, —CON-Alkyl- mit 1 bis 6 C-Atomen im Alkyl, —CON-Phenyl, —CON-Benzyl-,

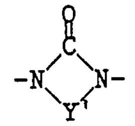 , worin Y' für 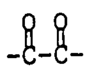, 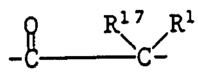 oder 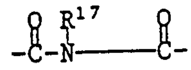

steht und $R^{17}$ ein Wasserstoffatom, $C_1$—$C_6$-Alkyl oder Phenyl bedeutet, lineares oder verzweigtes Alkylen mit 1 bis 3 C-Atomen, gegebenenfalls durch Cl oder F substituiertes Alkyliden mit 2 bis 12 C-Atomen, Cycloalkyliden mit 5 oder 6 Ringkohlenstoffatomen, Phenylen, Phenylendioxy, oder die Gruppe

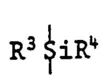, 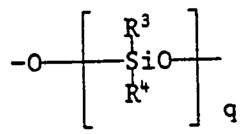 oder

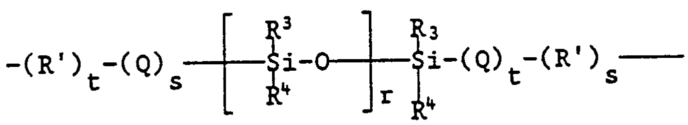 steht, worin $R^3$ und $R^4$ Alkyl oder Alkoxy mit 1 bis 6 C-Atomen, Phenyl, Benzyl, Phenyloxy oder Benzyloxy sind, r eine Zahl von 1 bis 10, t Null oder die Zahl 1 und s Null oder die Zahl 1 ist, und R'—O— oder —S— und Q $C_1$—$C_6$-Alkylen bedeuten, und q für eine Zahl von 1 bis 100 steht, $R^5$ und $R^6$ Alkyl, oder Alkoxy mit 1 bis 12 C-Atomen, Alkoxyalkyl mit 2 bis 12 C-Atomen, Cyclopentyl, Cyclohexyl oder Benzyl sind oder in den Formeln VIII oder VIIIa $R^5$ und $R^7$ in Nachbarstellung gebunden sind und zusammen Trimethylen oder Tetramethylen bedeuten, wobei $R^6$ auch ein Wasserstoffatom sein kann, $R^7$ und $R^8$ ein Wasserstoffatom sind oder unabhängig die Bedeutung von $R^5$ und $R^6$ haben, und $R^9$ und $R^{10}$ ein Wasserstoffatom sind, unabhängig die Bedeutung von $R^5$ und $R^6$ haben oder $R^7$ und $R^9$ in Formel VIIIa zusammen Trimethylen oder Tetramethylen sind.

11. Verfahren gemäss Anspruch 10, dadurch gekennzeichnet, dass man Polyimide verwendet, worin $R^5$ und $R^6$ in Formeln VIII, VIIIa und VIIIb Alkyl mit 1 bis 6 C-Atomen, besonders Methyl, Aethyl, n-Propyl oder Isopropyl sind.

12. Verfahren gemäss Anspruch 10, dadurch gekennzeichnet, dass man Polyimide verwendet, worin $R^{11}$ in Formel VIIIa —$CH_2$—, —O—, —CO— oder eine direkte Bindung darstellt.

13. Verfahren gemäss Anspruch 10, dadurch gekennzeichnet, dass man Polyimide verwendet, worin in Formel VIIIa die freien Bindungen in Parastellung zur $R^{11}$-Gruppe stehen.

14. Verfahren gemäss Anspruch 10, dadurch gekennzeichnet, dass man Polyimide verwendet, worin X in Formel I Resten der Formeln

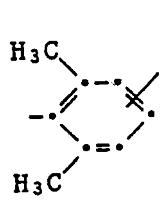 , 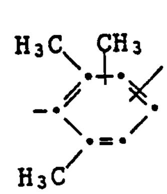 , 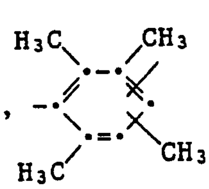 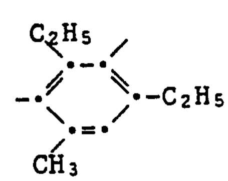

entspricht, worin die freien Bindungen in Meta- oder Parastellung zueinander stehen, oder der Formel

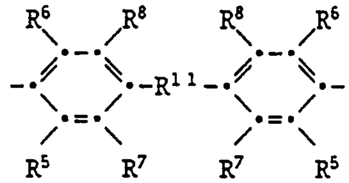

entspricht, worin $R^5$ und $R^6$ unabhängig Methyl, Aethyl, n-Propyl oder Isopropyl und $R^7$ und $R^8$ ein Wasserstoffatom sind oder die Bedeutung von $R^5$ haben oder $R^5$ und $R^7$ zusammen Tri- oder Tetramethylen bedeuten und $R^6$ und $R^8$ ein Wasserstoffatom sind, und $R^{11}$ eine direkte Bindung, $CH_2$ oder CO ist.

15. Verfahren gemäss Anspruch 1, dadurch gekennzeichnet, dass man Polyimide verwendet, worin X als substituierter aromatischer Rest der Formel IX entspricht,

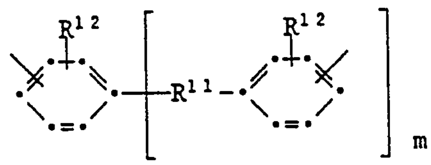 (IX),

worin m Null oder die Zahl 1 ist, die freien Bindungen in Meta- und bevorzugt in Orthostellung zur $R^{12}$-Gruppe gebunden sind, $R^{11}$ die gleiche Bedeutung wie in Formel VIIIa hat und $R^{12}$ die gleiche Bedeutung wie $R^5$ hat.

16. Verfahren gemäss Anspruch 1, dadurch gekennzeichnet, dass man Polyimide verwendet, worin Q' in Formel III ein dreiwertiger Rest der Formeln

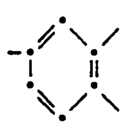 , 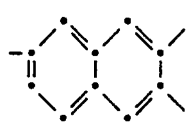 oder 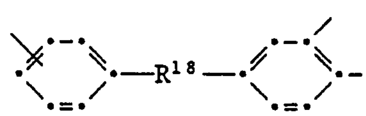

ist, worin die freie Bindung in Meta- oder Parastellung zur $R^{18}$-Gruppe gebunden ist und $R^{18}$ eine direkte Bindung, $—CH_2—$, —O—, —S—oder —CO— ist.

17. Verfahren gemäss Anspruch 1, dadurch gekennzeichnet, dass man Polyimide verwendet, worin X' in Formel II unsubstituiertes oder durch Halogen oder $C_1—C_4$-Acyl substituiertes Phenylen oder Bisphenylen der Formel

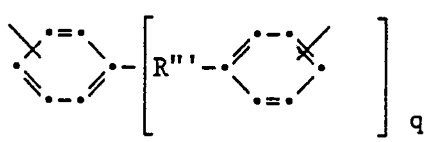

ist, worin q Null oder die Zahl 1 ist und R''' eine direkte Bindung, —O—, —S—, —SS—, —SO—, $—SO_2—$, —CO—,

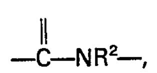

—COO—, $—NR^2—$, —NH—, —CONH—, $SiR^3R^4—$ oder $—OSi(R^3R^4)O$-bedeutet und $R^2$, $R^3$ und $R^4$ $C_1—C_6$-Alkyl, Phenyl oder Benzyl und $R^3$ und $R^4$ auch $C_1—C_6$-Alkoxy, Phenyloxy oder Benzyloxy sind.

18. Tetracarbonsäuren der Formel XII

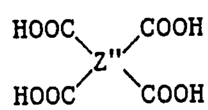 XII ,

worin Z'' ein Rest der Formeln XIIIa bis XIIIc

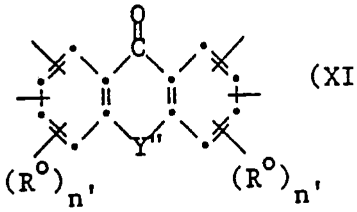 (XIIIa) , 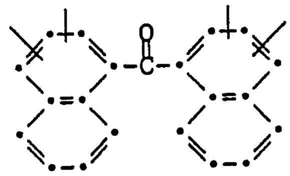 (XIIIb),

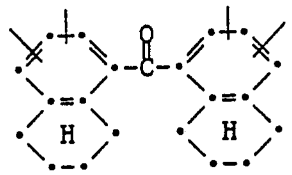 (XIIIc)

ist, in denen die freien Bindungen in Orthostellung zueinander gebunden sind, und Y'' eine direkte Bindung, $-CH_2-$, $-CH_2-CH_2-$, $-O-$, $-S-$, $-SO-$, $-SO_2-$, $-NR-$ oder $-CRR^1-$ bedeutet, worin R ein Wasserstoffatom, $C_1-C_{10}$-Alkyl, Phenyl, Naphthyl oder Phenyl($C_aH_{2a}$)- mit a = 1—4 und $R^1$ die Bedeutung von R mit der Ausnahme der Bedeutung eines Wasserstoffatomes hat und R° für $C_1-C_{10}$-Alkyl, Halogen, $-CN$, $-NO_2$, $C_1-C_{12}$-Alkoxy, Phenoxy, Naphthoxy oder Phenyl($C_aH_{2a}$)- mit a = 1—4 steht, und n' 0, 1 oder 2 ist, sowie die Säurederivate.

19. Tetracarbonsäuren der Formeln XIIIa bis XIIIc gemäss Anspruch 20, worin die freien Bindungen in Meta- und Parastellung zur CO-Gruppe gebunden sind, n' Null ist und Y'' für eine direkte Bindung, $-CH_2-$, $-O-$ oder $-S-$ steht.

## Revendications

1. Procédé pour préparer des pellicules protectrices ou des images photographiques en relief à partir de polyimides, procédé caractérisé en ce qu'on expose, sur toute la surface ou sous un photomasque (masque de photo-réserve) un matière revêtue, constituée d'une matière de support sur une face au moins de laquelle est appliquée une couche d'un homopolyimide ou d'un copolyimide photoréticulable, dérivant d'au moins un acide tétracarboxylique aromatique et d'au moins une diamine, les polyimides contenant 0,1 à 100 moles % d'au moins un élément de structure de formule I:

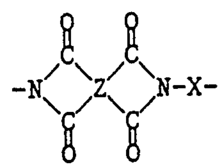 (I)

et 99,9 à 0 mole % d'au moins un élément de structure répondant aux formules II et/ou III:

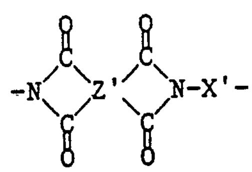 (II),

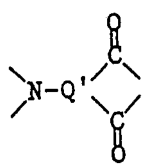 (III)

formules dans lesquelles:

Z représente au moins un reste tétravalent répondant aux formules IV, V , VI ou VII:

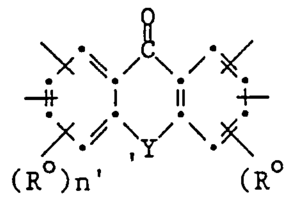 (IV), 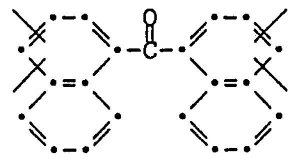 (V)

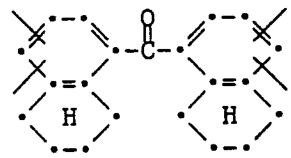 (VI) 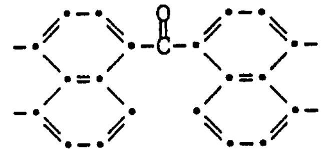 (VII)

dans lesquelles les liaisons libres se trouvent en position ortho l'une rapport à l'autre, et Y représente une liaison directe, $—CH_2—$, $—(CH_2)_2—$, —O—, —S—, —SO—, $—SO_2—$, —CO—, —NR— ou $—CRR^1—$, le symbole R représentant un atome d'hydrogène, un groupe alkyle en $C_1$ à $C_{10}$, un groupe phenyle, naphtyle ou phényle $(C_aH_{2a})$-, dans lequel a vaut 1 à 4, et R' a le sens de R, à l'exclusion du sens d'un atome d'hydrogène; R° représente un groupe alkyle en $C_1$ à $C_{10}$, halogéno, —CN—, $—NO_2$, un groupe alcoxy en $C_1$ à $C_{12}$, phénoxy, naphtoxy ou phényl-$(C_aH_{2a})$-, où a vaut 1 à 4, n' est nul ou vaut 1 ou 2,

X représente un reste aliphatique divalent, non substitué ou substitué (qui peut être interrompu par des hétéro-atomes, par des groupes aromatiques, hétérocycliques ou cycloaliphatiques), un reste hétérocyclique, cycloaliphatique ou araliphatique non substitué ou substitué, un reste aromatique, dans lequel deux noyaux aryles sont reliés par l'intermédiaire d'un groupe aliphatique, ou un reste aromatique (substitué par au moins un groupe alkyle, cycloalkyle, alcoxy, alcoxyalkyle, alkylthio, alkylthioalkyle, hydroxyalkyle, hydroxyalcoxy, hydroxyalcoxythio, aralkyle, ou bien 2 atomes de C vicinaux du reste aromatique sont substitués par un groupe alkylène,

Q' represente un reste aromatique trivalent,

Z' a le même sens que Z, ou bien il représente un reste organique tétravalent, qui diffère de Z, et

X' est un reste divalent, différent de X, d'une diamine organique,

et Z peut représenter également dans la formule I des restes benzophénones plurivalents, lorsque des éléments de structure de formule III sont contenus, et l'on enlève ensuite à l'aide d'un solvant les parties non touchées par la lumière.

2. Procédé selon la revendication 1, caractérisé en ce qu'on utilise des polyimides dans lesquels, dans la formule IV, Y représente une liaison directe, $—CH_2—$, —O—, —S— ou —CO—.

3. Procédé selon la revendication 1, caractérisé en ce qu'on utilise des polyimides pour lesquels, dans les formules IV à VII, les liaisons libres sont en position méta et para par rapport au groupe CO.

4. Procédé selon la revendication 1, caractérisé en ce qu'on utilise des polyimides dans lesquels le substituant du reste aromatique contient, quand il est un groupe alkyle ou alcoxy, 1 à 20 atomes de carbone, il contient 2 à 12 atomes de carbone quand il est un groupe alcoxyalkyle, il contient 5 ou 6 atomes de carbone de noyaux quand il est un groupe cycloalkyle et il contient 3 ou 4 atomes de carbone quand il est un groupe alkylène et, lorsqu'il est un groupe aralkyle, il est un groupe benzyle.

5. Procédé selon la revendication 4, caractérisé en ce qu'on utilise des polyimides dans lesquels le substituant alkyle comporte 1 à 4 atomes de carbone et est de préférence un groupe isopropyle ou éthyle et notamment un groupe méthyle.

6. Procédé selon la revendication 1, caractérisé en ce qu'on utilise des polyimides dans lesquels Z' contient 6 à 30 atomes de carbone lorsqu'il représente dans la formule II un reste aromatique différent de Z.

7. Procédé selon la revendication 6, caractérisé en ce qu'on utilise des polyimides dans le cas desquels, dans la formule II, Z' représente un reste de formule:

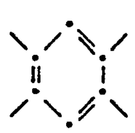 et en particulier : 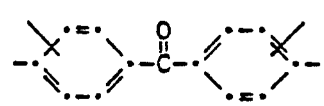

ou des mélanges de ces restes.

8. Procédé selon la revendication 1, caractérisé en ce qu'on utilise des polyimides dans lesquels 1, ou en particulier 2, substituant(s) ont ou sont fixés, dans le reste aromatique de la formule I, en position ortho par rapport à l'atome d'azote.

9. Procédé selon la revendication 1, caractérisé en ce qu'on utilise des polyimides dans lesquels, l'aspect représente un reste aliphatique, X de la formule I contient 2 à 30 atomes de carbone, quand il représente un reste cycloaliphatique il contient 5 à 8 atomes de carbone de noyaux, quand il représente un reste araliphatique il contient 7 à 30 atomes de carbone et, quand il représente un reste aromatique

substitué, il contient 7 à 30 atomes de carbone.

10. Procédé selon la revendication 1, caractérisé en ce qu'on utilise des polyimides dans lesquels X, quand il représente dans la formule I und reste aromatique substitué, répond aux formules VIII, VIIIa et/ou VIIIb:

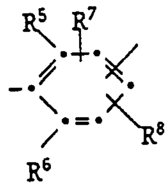 (VIII) 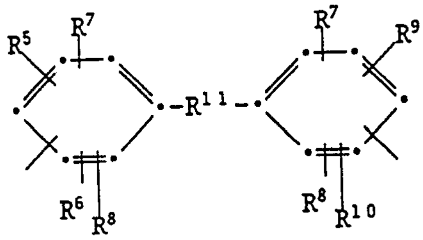 (VIIIa)

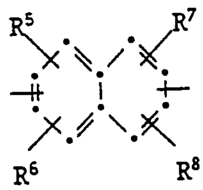 (VIIIb),

et, dans la formule VIII, les liaisons libres sont situées en position méta ou para l'une par rapport à l'autre, dans la formule VIIIa les liaisons libres sont de préférence en position méta ou para par rapport au groupe $R^{11}$ et $R^5$ et $R^6$, sont dans les deux positions en ortho par rapport à la liaison libre, et, dans la formule VIIIb, les liaisons libres sont en position 2, 3, 6 et 7 et $R^5$ et $R^6$ sont dans les deux positions en ortho des liaisons libres; $R^{11}$ représente une liaison directe, —O—, —S—, —SS—, —SO—, —$SO_2$—, —CO—, —COO—, —NH—,

un groupe —N(|)-alkyle

comportant 1 à 6 atomes de carbone dans le groupe alkyle, un groupe

—N(|)-phényle,

—N(|)-benzyle,

—CONH—, —CON-alkyle comportant 1 à 6 atomes de carbone dans le groupe alkyle, —CON-phényle, —CON-benzyle,

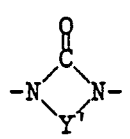 , où Y' représente 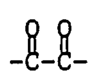, 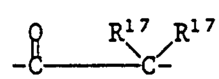 ou 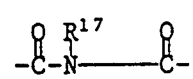

et $R^{17}$ représente un atome d'hydrogène, un groupe alkyle en $C_1$ à $C_6$ ou un groupe phényle, un groupe alkylène linéaire ou ramifié comportant 1 à 3 atomes de carbone, un groupe alkylidène ayant 2 à 12 atomes de carbone (éventuellement substitués par Cl ou F), un groupe cycloalkylidène comportant 5 ou 6 atomes de carbone de noyaux, un groupe phénylène, phénylènedioxy, ou le groupe:

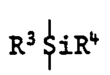 , 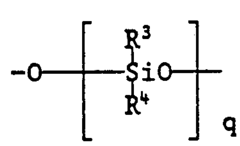 ou

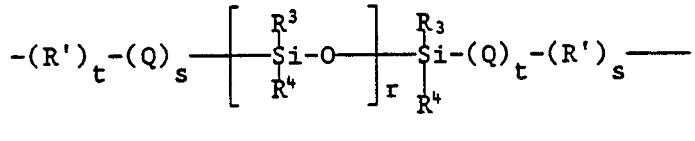

où $R^3$ et $R^4$ représentent chacun un groupe alkyle ou alcoxy ayant 1 à 6 atomes de carbone, un groupe phényle, benzyle, phényloxy ou benzyloxy; r est un nombre valant 1 à 10; o est nul ou vaut 1 et s est nul ou vaut 1; et R' représente —O— ou —S— et Q représente un groupe alkylène en $C_1$ à $C_6$, et q est un nombre valant 1 à 100; $R^5$ et $R^6$ représentent chacun un groupe alkyle ou alcoxy ayant 1 à 12 atomes de carbone, alcoxyalkyle ayant 2 à 12 atomes de carbone, cyclopentyle, cyclohexyle ou benzyle ou bien, dans les formules VIII ou VIIIa, $R^5$ et $R^7$ sont fixés en des positions voisines et forment ensemble un groupe triméthylène ou tétraméthylène, $R^5$ pouvant également représenter un atome d'hydrogène, $R^7$ et $R^8$ représentent chacun un atome d'hydrogène ou bien ils ont, indépendamment, le sens de $R^5$ et de $R^6$, et $R^9$ et $R^{10}$ représentent chacun un atome d'hydrogène, ils ont, indépendamment, le sens de $R^5$ et $R^6$ ou bien, dans la formule VIIIa, $R^7$ et $R^9$ forment ensemble un groupe triméthylène ou tétraméthylène.

11. Procédé selon la revendication 10, caractérisé en ce qu'on utilise des polyimides dans lesquels, dans les formules VIII, VIIIa et VIIIb, les symboles $R^5$ et $R^6$ rprésentent chacun un groupe alkyle ayant 1 à 6 atomes de carbone, notamment un groupe méthyle, éthyle, propyle ou isopropyle.

12. Procédé selon la revendication 10, caractérisé en ce qu'on utilise des polyimides dans lesquels, dans les formule VIIIa, $R^{11}$ représente $—CH_2—$, —O—, —CO— ou une liaison directe.

13. Procédé selon la revendication 10, caractérisé en ce qu'on utilise des polyimides dans lesquels, les liaisons libres présentes dans la formule VIIIa sont en position para par rapport au groupe $R^{11}$.

14. Procédé selon la revendication 10, caractérisé en ce qu'on utilise des polyimides, dans lesquels X représente dans la formule I des restes répondant aux formules:

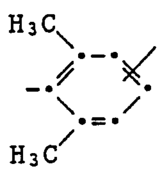 , 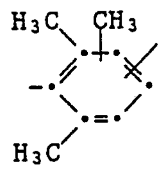 , 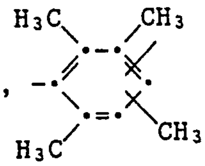 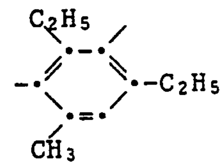

où les liaisons libres sont en position méta ou para l'une par rapport à l'autre, ou bien X représente le reste de formule:

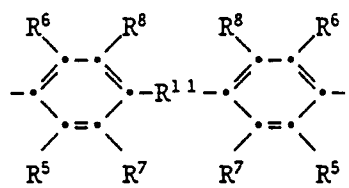

dans laquelle $R^5$ et $R^6$ représentent, indépendamment l'un de l'autre, un groupe méthyle, éthyle, propyle ou isopropyle, et $R^7$ et $R^8$ représentent chacun un atome d'hydrogène ou ont le sens de $R^5$, ou bien $R^5$ et $R^7$ forment ensemble un groupe triméthylène ou tétraméthylene, et $R^6$ et $R^8$ représentent chacun un atome d'hydrogène, et $R^{11}$ est une liaison directe ou représente $CH_2$ ou CO.

15. Procédé selon la revendication 1, caractérisé en ce qu'on utilise des polyimides dans lesquels, lorsqu'il représente un reste aromatique substitué, X répond à la formule IX:

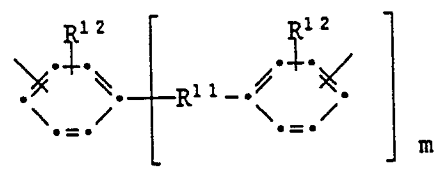 (IX),

dans laquelle m est nul ou vaut 1, les liaisons libres sont en position méta et de préférence en position ortho par rapport au groupe $R^{12}$, $R^{11}$ a le même sens que dans la formule VIIIa et $R^{12}$ a le même sens que $R^5$.

16. Procédé selon la revendication 1, caractérisé en ce qu'on utilise des polyimides dans lesquels Q' représente dans la formule III un reste trivalent répondant aux formules:

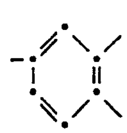 , 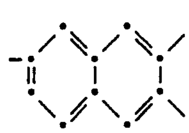 ou 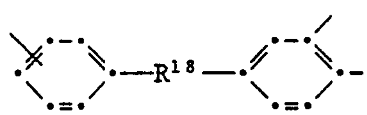

où la liaison libre se trouve en position méta ou para par rapport au groupe $R^{18}$, et $R^{18}$ représente une liaison directe, $—CH_2—$, —O—, —S— ou —CO—.

17. Procédé selon la revendication 1, caractérisé en ce qu'on utilise des polyimides dans lesquels, dans

la formule II, X' représente un groupe phénylène ou bisphénylène (non substitué ou substitué par de l'halogène ou par un groupe acyle en $C_1$ à $C_4$) de formule:

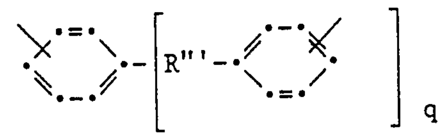

dans laquelle q est nul ou vaut 1 et R''' représente une liaison directe, —O—, —S—, —SS—, —SO—, $—SO_2—$, —CO—,

||
$—C—NR^2—$,

—COO—, $—NR^2—$, —NH—, —CONH—, $—SiR^3R^4—$ ou $OSi(R^3R^4(O—$, et les symboles $R^2$, $R^3$ et $R^4$ représentent chacun un groupe alkyle en $C_1$ à $C_6$, phényle, ou benzyle, et $R^3$ et $R^4$ peuvent également représenter un groupe alcoxy en $C_1$ à $C_6$, phényloxy ou benzyloxy.

18. Acides tétracarboxyliques de formule XIII:

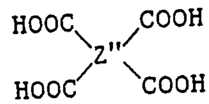

XII ,

dans laquelle Z'' représente un reste répondant aux formules XIIIa à IIIc:

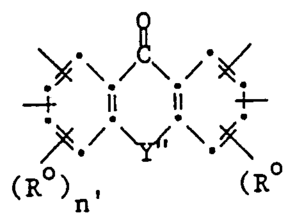 (XIIIa) , 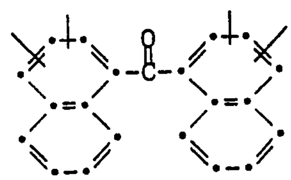 (XIIIb),

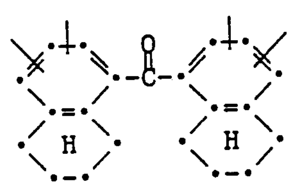 (XIIIc)

dans lesquelles les liaisons libres sont en position ortho l'une par rapport à l'autre, et Y'' représente une liaison directe ou un groupe $—CH_2—$, $—CH_2—CH_2—$, —O—, —S—, —SO—, $—SO_2—$, —NR— ou $—CRR^1—$, où R représente un atome d'hydrogène, un groupe alkyle en $C_1$ à $C_{10}$, un groupe phényle, naphtyle ou phényl $(C_aH_{2a})$-, dans lequel a vaut 1 à 4, et $R^1$ a le sens de R à l'exclusion du sens d'un atome d'hydrogène, et R° représente un groupe alkyle en $C_1$ à $C_{10}$, halogéno, —CN, $—NO_2$, -alkoxy en $C_1$ à $C_{12}$, phénoxy, naphtoxy ou phényl-$(C_aH_{2a})$-, avec a valant 1 à 4, et n' vaut 0, 1 ou 2, ainsi que les dérivés des acides.

19. Acides tétracarboxyliques répondant aux formules XIIIa à XIIIc selon la revendication 20, dans lesquelles les liaisons libres sont en position méta et para par rapport au groupe CO, n' est nul et Y'' représente une liaison directe, $—CH_2—$, —O— ou —S—.

**Claims**

1. A process for the production of protective films or photographic relief images from polyimides, which comprise irradiating the area of a coated material or irradiating it under a photomask and then removing the non-exposed portions with a solvent, the coated material comprising a carrier material onto which has been applied on at least one surface a layer of a photo crosslinkable homo- or copolyimide of at least one aromatic tetracarboxylic acid and at least one diamine, the polyimides containing 0.1 to 100 mol% of at least one structural element of the formula I

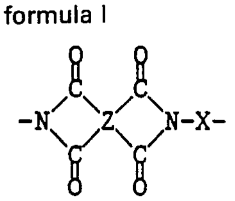

(I)

and 99.9 to 0 mol% of at least one structural element of the formulae II and/or III

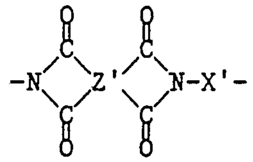

(II),

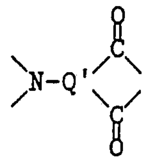

(III)

in which Z is at least one tetravalent radical of the formula IV, V, VI or VII

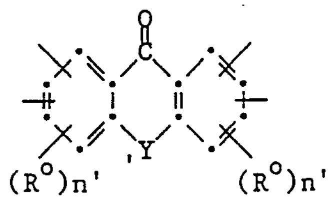

(IV),

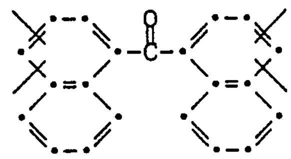

(V)

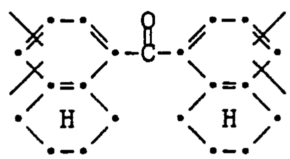

(VI)

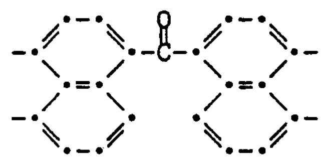

(VII)

in which the free bonds are in the ortho-position relative to one another and Y is a direct bond, —$CH_2$—, —$(CH_2)_2$—, —O—, —S—, —SO—, —$SO_2$—, —CO—, —NR— or —$CRR^1$—, in which R is a hydrogen atom, $C_1$—$C_{10}$alkyl, phenyl, naphthyl or phenyl($C_aH_{2a}$)-, where a is 1—4, and $R^1$ is R, with the exception of a hydrogen atom, R° is $C_1$—$C_{10}$alkyl, halogen, —CN, —$NO_2$, $C_1$—$Cl_2$alkoxy, phenoxy, naphthoxy or phenyl-($C_aH_{2a}$)-, where a is 1—4 n′ is zero or the number 1 or 2, X is an unsubstituted or substituted divalent aliphatic radical, which can be interrupted by heteroatoms or aromatic, heterocyclic or cycloaliphatic groups, a substituted or unsubstituted heterocyclic, cycloaliphatic or araliphatic radical, an aromatic radical, in which two aryl nuclei are linked via an aliphatic group, or an aromatic radical which is substituted by at least one alkyl group, cycloalkyl group, alkoxy group, alkoxyalkyl group, alkylthio group, alkylthioalkyl group, hydroxyalkyl group, hydroxyalkoxy group, hydroxyalkylthio group, aralkyl group or, in the case of two adjacent C atoms of the aromatic radical, Q′, is a trivalent aromatic radical, Z′ has the same meaning as Z or is a tetravalent organic radical other than Z and X′ is the divalent radical, other than X, of an organic diamine, and in which Z in formula I can also be a tetravalent benzophenone radical if structural elements of the formula III are present.

2. A process according to claim 1, which comprises using a polyimide in which Y in formula IV is a direct bond, —$CH_2$—, —O—, —S— or —CO—.

3. A process according to claim 1, which comprises using a polyimide in which the free bonds in formulae IV to VII are in the meta- or para-position relative to the CO group.

4. A process according to claim 1, which comprises using a polyimide in which the substituent of the aromatic radical contains 1 to 20 C atoms as alkyl or alkoxy, 2 to 12 C atoms as alkoxyalkyl, 5 or 6 ring carbon atoms as cycloalkyl and 3 or 4 C atoms as alkylene and is benzyl as aralkyl.

5. A process according to claim 4, which comprises using a polyimide in which the substituent is alkyl with 1 to 4 C atoms, preferably isopropyl, ethyl or, in particular, methyl.

6. A process according to claim 1, which comprises using a polyimide in which in formula 2 an aromatic radical Z′ other than Z contains 6 to 30 C atoms.

7. A process according to claim 6, which comprises using a polyimide in which Z′ in formula 2 is a radical of the formula

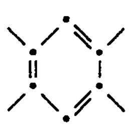

or, in particular

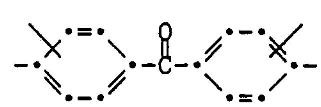

or a mixture thereof.

8. A process according to claim 1, which comprises using a polyimide in which one or, in particular, two substituents in the aromatic radical X in formula 1 are bonded in the ortho-position relative to the N atom.

9. A process according to claim 1, which comprises using a polyimide in which X in formula 1 contains 2 to 30 C atoms as an aliphatic radical, 5 to 8 ring C atoms as a cycloaliphatic radical, 7 to 30 C atoms as an araliphatic radical and 7 to 30 C atoms as a substituted aromatic radical.

10. A process according to claim 1, which comprises using a polyimide in which in formula 1 a substituted aromatic radical X has the formulae VIII, VIIIa and/or VIIIb

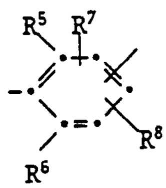 (VIII) 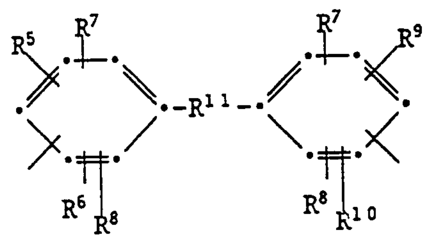 (VIIIa)

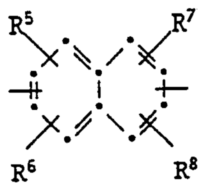 (VIIIb),

in which in formula VIII the free bonds are in the meta- or para-position relative to one another, in formula VIIIa the free bonds are preferably in the meta- or para-position relative to the $R^{11}$ group and $R^5$ and $R^6$ are bonded in the two ortho-positions relative to the free bond, and in formula VIIIb the free bonds are in the 2-, 3-, 6- and 7-position and $R^5$ and $R^6$ are in the two ortho-positions relative to the free bonds, $R^{11}$ is a direct bond, —O—, —S—, —SS—, —SO—, —$SO_2$—, —CO—, —COO—, —NH—,

—N-alkyl

with 1 to 6 C atoms in the alkyl,

—N-phenyl,

—N-benzyl,

—CONH—, —CON-alkyl- with 1 to 6 C atoms in the alkyl, —CON-phenyl, —CON-benzyl-,

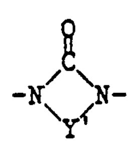 , **in which Y' is** 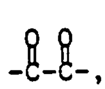, 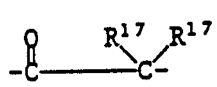 **or** 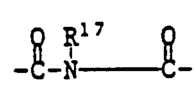

and $R^{17}$ is a hydrogen atom, $C_1$—$C_6$alkyl or phenyl, linear or branched alkylene with 1 to 3 C atoms, alkylidene with 2 to 12 C atoms which is unsubstituted or substituted by Cl or F, cycloalkylidene with 5 or 6 ring carbon atoms, phenylene, phenylenedioxy or the group

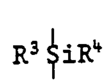, 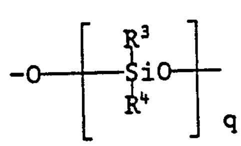 or

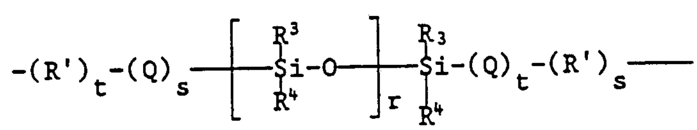

is, in which $R^3$ and $R^4$ are alkyl or alkoxy with 1 to 6 C atoms, phenyl, benzyl, phenoxy or benzyloxy, r is a number from 1 to 10, t is zero or the number 1 and s is zero or the number 1, and R' is —O— or —S— and Q is $C_1$—$C_6$alkylene, and q is a number from 1 to 100, $R^5$ and $R^6$ are alkyl or alkoxy with 1 to 12 C atoms, alkoxyalkyl with 2 to 12 C atoms, cyclopentyl, cyclohexyl or benzyl, or in the formula VIII or VIIIa $R^5$ and $R^7$ are bonded in adjacent positions and together are trimethylene or tetramethylene, in which $R^6$ can also be a hydrogen atom, $R^7$ and $R^8$ are a hydrogen atom or independently have the meaning of $R^5$ and $R^6$, and $R^9$ and $R^{10}$ are a hydrogen atom, or independently have the meaning of $R^5$ and $R^6$, or $R^7$ and $R^9$ in formula VIIIa together are trimethylene or tetramethylene.

11. A process according to claim 10, which comprises using a polyimide in which $R^5$ and $R^6$ in formulae VIII, VIIIa, and VIIIb are alkyl with 1 to 6 C atoms, in particular methyl, ethyl, n-propyl or isopropyl.

12. A process according to claim 10, which comprises using a polyimide in which $R^{11}$ in formula VIIIa is —$CH_2$—, —O—, —CO— or a direct bond.

13. A process according to claim 10, which comprises using a polyimide in which the free bonds in formula VIIIa are in the para-position relative to the $R^{11}$ group.

14. A process according to claim 10, which comprises using a polyimide in which X in formula I is a radical of the formula

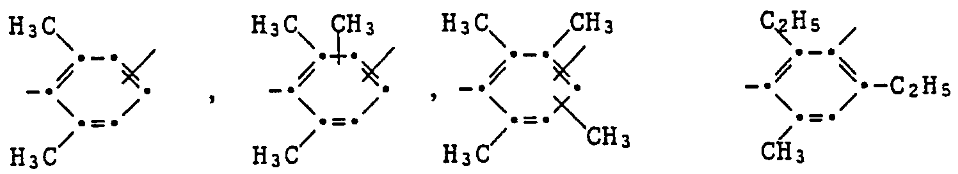

in which the free bonds are in the meta- or para-position relative to one another, or of the formula

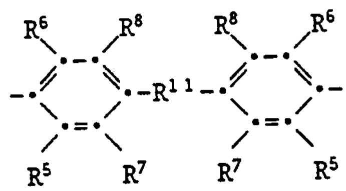

in which $R^5$ and $R^6$ independently are methyl, ethyl, n-propyl or isopropyl and $R^7$ and $R^8$ are a hydrogen atom or have the meaning of $R^5$, or $R^5$ and $R^7$ together are tri- or tetramethylene and $R^6$ and $R^8$ are a hydrogen atom, and $R^{11}$ is a direct bond, $CH_2$ or CO.

15. A process according to claim 1, which comprises using a polyimide in which a substituted aromatic radical X has the formula IX

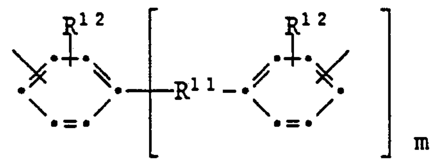

(IX),

in which m is zero or the number 1, the free bonds are in the meta- or, preferably, in the ortho-position relative to the $R^{12}$ group, $R^{11}$ is as defined for formula VIIIa and $R^{12}$ has the same meaning as $R^5$.

16. A process according to claim 1, which comprises using a polyimide in which Q', in formula III is a trivalent radical of the formula

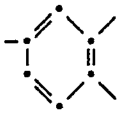

,

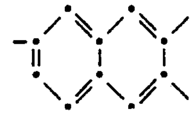

or

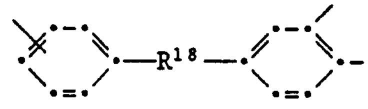

in which the free bond is in the meta- or para-position relative to the $R^{18}$ group and $R^{18}$ is a direct bond, —$CH^2$—, —O—, —S— or —CO—.

17. A process according to claim 1, which comprises using a polyimide in which X' in formula II is phenylene which is unsubstituted or substituted by halogen or $C_1$—$C_4$acyl, or bisphenylene of the formula

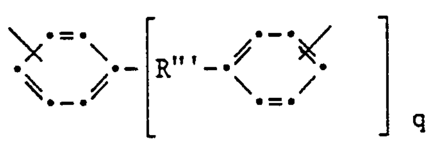

in which q is zero or the number 1 and R''' is a direct bond, —O—, —S—, —SS—, —SO—, —SO$_2$—, —CO—,

$-\overset{\|}{C}-NR^2-$,

—COO—, —NR$^2$—, —NH—, —CONH—, —SiR$^3$R$^4$— or —OSi(R$^3$R$^4$(O—, and R$^2$, R$^3$ and R$^4$ are $C_1$—$C_6$alkyl, phenyl or benzyl and R$^3$ and R$^4$ are also $C_1$—$C_6$alkoxy, phenoxy or benzyloxy.

18. A tetracarboxylic acid of the formula XII

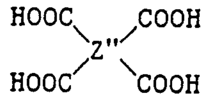

XII ,

in which Z'' is a radical of the formulae XIIIa to XIIIc

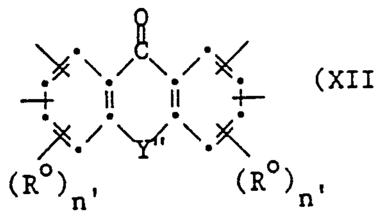

(XIIIa) ,

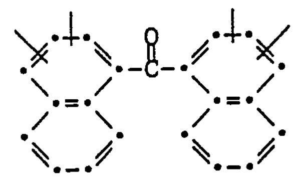

(XIIIb),

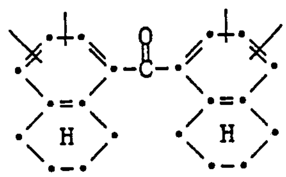

(XIIIc)

in which the free bonds are in the ortho-position relative to one another and Y'' is a direct bond, —CH$_2$—, —CH$_2$—CH$_2$—, —O—, —S—, —SO—, —SO$_2$—, —NR— or —CRR$^1$—, in which R is a hydrogen atom, $C_1$—$C_{10}$alkyl, phenyl, naphthyl or phenyl-($C_aH_{2a}$)-, where a is 1—4 and R$^1$ has the meaning of R, with the exception of a hydrogen atom, and R° is $C_1$—$C_{10}$alkyl, halogen, —CN, —NO$_2$, $C_1$—$C_{12}$alkoxy, phenoxy, naphthoxy or phenyl($C_aH_{2a}$)-, where a is 1—4, and n' is 0, 1 or 2, and an acid derivative.

19. A tetracarboxylic acid of the formula XIIIa to XIIIc according to claim 20, wherein the free bonds are in the meta- or para-position relative to the CO group, n' is zero and Y'' is a direct bond, —CH$_2$—, —O— or —S—.